# EUROPEAN PATENT SPECIFICATION

(11) **EP 4 299 108 B1**
(45) Date of publication and mention of the grant of the patent: **25.12.2024**
(21) Application number: 23182166.1
(22) Date of filing: 28.06.2023
(51) Int. Cl.: A61N 1/36, A61B 5/00, A61N 1/375, A61N 1/378, A61N 1/372

(54) **COORDINATING USE OF POWER-SAVING BATTERY SWITCH HARDWARE FEATURE BY MULTIPLE FIRMWARE FEATURES**
KOORDINATION DER VERWENDUNG EINER ENERGIESPARENDEN BATTERIESCHALTER-HARDWAREFUNKTION DURCH MEHRERE FIRMWARE-FUNKTIONEN
COORDINATION DE L'UTILISATION D'UNE CARACTÉRISTIQUE MATÉRIELLE DE COMMUTATEUR DE BATTERIE À ÉCONOMIE D'ÉNERGIE PAR DE MULTIPLES CARACTÉRISTIQUES DE MICROLOGICIEL

(30) Priority: 28.06.2022 US 202263367177 P; 02.06.2023 US 202318328248
(43) Date of publication of application: 03.01.2024
(73) Proprietor: Medtronic, Inc., Minneapolis, MN 55432 (US)
(72) Inventor: Shamus, Jake D., Minneapolis, 55432 (US); Voje, Eric, Minneapolis, 55432 (US); Skarie, Laura Ann, Minneapolis, 55432 (US); Erler, James E., Minneapolis, 55432 (US); Vasoli, Maile, Minneapolis, 55432 (US); Simanovich, Igor, Minneapolis, 55432 (US); Shongwe, Mandla, Minneapolis, 55432 (US)
(74) Representative: Zimmermann & Partner Patentanwälte mbB

(56) References cited:
- US-A1- 2005 107 841
- US-A1- 2008 132 974
- US-A1- 2020 230 406
- US-A1- 2021 275 808
- US-B2- 11 266 837
- US-B2- 9 744 347

## Description

This application claims the benefit of U.S. Provisional Patent Application 63/367,177, filed on June 28, 2022.

### TECHNICAL FIELD

The disclosure relates to power saving features in a device, such as a device configured to deliver therapy to a patient.

### BACKGROUND

Disease, age, and injury may impair physiological functions of a patient. In some situations, the physiological functions are completely impaired. In other examples, the physiological function may operate sufficiently at some times or under some conditions and operate inadequately at other times or at other conditions. In one example, bladder dysfunction, such as overactive bladder, urgency, or urinary incontinence, is a problem that may afflict people of all ages, genders, and races. Various muscles, nerves, organs and conduits within the pelvic floor cooperate to collect, store and release urine. A variety of disorders may compromise urinary tract performance, and contribute to an overactive bladder, urgency, or urinary incontinence that interferes with normal physiological function. Many of the disorders may be associated with aging, injury or illness.

Urinary incontinence may include urge incontinence and stress incontinence. In some examples, urge incontinence may be caused by disorders of peripheral or central nervous systems that control bladder micturition reflexes. Some patients may also suffer from nerve disorders that prevent proper triggering and operation of the bladder, sphincter muscles or nerve disorders that lead to overactive bladder activities or urge incontinence. In some cases, urinary incontinence may be attributed to improper sphincter function, either in the internal urinary sphincter or external urinary sphincter. Documents US 2008/132974 A1 and US 9744347 B2 relate to stimulation devices.

### SUMMARY

In general, the disclosure is directed to devices, systems, and techniques for controlling power delivery in a device, such as an implantable medical device (IMD), like an implantable stimulation device (an implantable neurostimulator). For example, this disclosure describes examples of devices, systems, and techniques for reducing power consumption in battery powered devices, such as an IMD. While primarily discussed herein as being used in an IMD, the techniques of this disclosure may be used in any device having a battery as a power source.

For many stimulation therapies, the tonic, patterned, pulsed, or cycled stimulation pulses have periods of time where the delivery of stimulation to the body is withheld. During certain time periods where therapy is withheld, the majority of the electronics of the IMD may be disconnected from the battery power, or any power source derived from the battery. Such a state may be referred to herein as a hibernation mode. While power is disconnected from the majority of the electronics of the IMD, power may remain connected to some portion of the IMD, such as power domain circuitry which may close a battery switch to restore power to the rest of the device. Such power domain circuitry may have a firmware counterpart that may moderate, mediate, or arbitrate requests to open the battery switch or keep the battery switch closed. The power domain firmware may control the position of the battery switch to open or keep closed based on requests received by the power domain firmware from a plurality of firmware modules within the IMD. The plurality of firmware modules may be referred to herein as modules as at least two of the plurality of firmware modules may send requests on behalf of corresponding hardware or for themselves related to whether to open the battery switch or keep the battery switch closed. In some examples, even though referred to as a plurality of firmware modules, the plurality of firmware modules may be implemented as a single piece of firmware. In some examples, the single piece of firmware may include the power domain firmware. The power domain firmware may moderate, mediate, or arbitrate the requests such that the battery switch is only open when each of the requests received by the power domain firmware indicates that the battery switch may be opened.

For implantable stimulators that are battery powered, the energy drain of the battery is from a combination of the stimulation current delivered to the nervous system and the energy required to operate the supporting electronics. Supporting electronics may include processor circuitry such as a microprocessor, telemetry circuitry, sensors, timing circuitry, and the like. In some cases, the supporting electronics can drain the battery as much as the stimulation current. Hence, by entering the hibernation mode, a large portion of the battery drain may be eliminated, for example, when the stimulation therapy is turned off, largely extending the battery life of the stimulator and/or increasing the recharge interval (e.g., the length of time between recharges).

In one example, the disclosure is directed to an implantable neurostimulator including: a battery configured to provide power to the implantable neurostimulator; a battery switch configured to open and remove power from one or more components of the implantable neurostimulator, or close to provide power to each component of the implantable neurostimulator requiring power to operate; and processing circuitry configured to: execute a plurality of firmware modules that are configured to perform respective functions of the implantable neurostimulator, at least two of the plurality of firmware modules being configured to determine whether power is needed for a corresponding hardware component or to perform the respective function during a respective time period and to generate and transmit one or more respective requests based on the determination of whether power is needed for the corresponding hardware component or to perform the respective function during the respective time period; and execute power domain firmware that configures the processing circuitry to: receive the one or more respective requests; determine whether to open the battery switch or to keep the battery switch closed in response to the one or more respective requests; and control the battery switch to open or keep closed in response to the determination.

In another example, the disclosure is directed to a method including receiving, by power domain firmware executing on processing circuitry and from at least two of a plurality of firmware modules executing on processing circuitry and configured to determine whether power is needed for a respective corresponding hardware component or to perform a respective function of an implantable neurostimulator during a respective time period and to generate and transmit one or more respective requests based on the determination of whether power is needed for the corresponding hardware component or to perform the respective function during the respective time period, the one or more respective requests; determining, by the power domain firmware, whether to open a battery switch or to keep the battery switch closed in response to the one or more respective requests; and controlling, by the power domain firmware, the battery switch to open or keep closed in response to the determination.

In another example, the disclosure is directed to a non-transitory computer-readable storage medium including instructions including a plurality of firmware modules and power domain firmware, which when executed, cause processing circuitry of an implantable neurostimulator to: perform respective functions of the implantable neurostimulator; determine whether power is needed for a corresponding hardware component or to perform the respective function during a respective time period; generate and transmit one or more respective requests based on the determination of whether power is needed for the corresponding hardware component or to perform the respective function during the respective time period; receive the one or more respective requests; determine whether to open a battery switch or to keep the battery switch closed in response to the one or more respective requests; and control the battery switch to open or keep closed in response to the determination.

In another example, the disclosure is directed to an implantable neurostimulator including: means for receiving, by power domain firmware executing on processing circuitry and from at least two of a plurality of firmware modules executing on processing circuitry and configured to determine whether power is needed for a respective corresponding hardware component or to perform a respective function of the implantable neurostimulator during a respective time period and to generate and transmit one or more respective requests based on the determination of whether power is needed for the corresponding hardware component or to perform the respective function during the respective time period, the one or more respective requests; means for determining, by the power domain firmware, whether to open a battery switch or to keep the battery switch closed in response to the one or more respective requests; and means for controlling, by the power domain firmware, the battery switch to open or keep closed in response to the determination.

An example implantable neurostimulator includes a battery configured to provide power to the implantable neurostimulator, a battery switch configured to open or close, and a plurality of firmware modules. At least two of the plurality of firmware modules are configured to generate and transmit one or more respective requests. The implantable neurostimulator also includes power domain firmware configured to receive the one or more respective requests, determine whether to open in response to the one or more respective requests, and control the battery switch to open in response to the determination.

An example implantable neurostimulator comprises: a battery configured to provide power to the implantable neurostimulator; a battery switch configured to open and remove power from one or more components of the implantable neurostimulator, or close to provide power to each component of the implantable neurostimulator requiring power to operate; and processing circuitry configured to: execute a plurality of firmware modules that are configured to perform respective functions of the implantable neurostimulator, at least two of the plurality of firmware modules being configured to determine whether power is needed for a corresponding hardware component or to perform the respective function during a respective time period and to generate and transmit one or more respective requests based on the determination of whether power is needed for the corresponding hardware component or to perform the respective function during the respective time period; and execute power domain firmware that configures the processing circuitry to: receive the one or more respective requests; determine whether to open the battery switch or to keep the battery switch closed in response to the one or more respective requests; and control the battery switch to open or keep closed in response to the determination.

The details of one or more examples are set forth in the accompanying drawings and the description below. Other features, objects, and advantages of the disclosure will be apparent from the description and drawings, and from the claims.

The above summary is not intended to describe each illustrated example or every implementation of the present disclosure. The present invention is set out in the appended claims.

### BRIEF DESCRIPTION OF DRAWINGS

FIG. 1A is a schematic diagram illustrating an example leadless neurostimulation device.
FIG. 1B is a conceptual diagram illustrating a leg having a leadless neurostimulation device implanted therein near a tibial nerve.
FIG. 1C is a conceptual diagram illustrating an example system that manages delivery of neurostimulation to a patient to manage bladder dysfunction, such as overactive bladder, urgency, or urinary incontinence.
FIG. 2 is a block diagram illustrating an example configuration of an implantable medical devices (IMD).
FIG. 3 is a block diagram illustrating an example configuration of an external programmer.
FIG. 4 is a block diagram illustrating an example neurostimulation device having a plurality of firmware modules according to the techniques of this disclosure.
FIG. 5 is a conceptual diagram illustrating example requests which a plurality of firmware modules may send to power domain firmware.
FIG. 6 is a flow diagram illustrating an example technique for controlling power delivery in a device according to one or more aspects of this disclosure.
FIGS. 7A-7D are flow diagrams illustrating example techniques for opening a battery switch according to one or more aspects of this disclosure.
FIGS. 8A-8B are flow diagrams illustrating example techniques for recovering after closing a battery switch according to one or more aspects of this disclosure.

### DETAILED DESCRIPTION

The disclosure is directed to devices, systems, and techniques for reducing current draw or power drain of a device, such as an IMD. The techniques of this disclosure may increase the battery life and/or the recharge interval (e.g., the time between recharges) of a device implementing such techniques. In some examples, these techniques may be used with a neurostimulator that may provide therapy for a variety of dysfunctions, diseases or disorders. For purposes of illustration, but without limitation, use of the techniques of this disclosure will be described below with respect to bladder dysfunction. Bladder dysfunction generally refers to a condition of improper functioning of the bladder or urinary tract, and may include, for example, an overactive bladder, urgency, or urinary incontinence. Overactive bladder (OAB) is a patient condition that may include symptoms, such as urgency, with or without urinary incontinence. Urgency is a sudden, compelling urge to urinate, and may often, though not always, be associated with urinary incontinence. Urinary incontinence refers to a condition of involuntary loss of urine, and may include urge incontinence, stress incontinence, or both stress and urge incontinence, which may be referred to as mixed urinary incontinence. As used in this disclosure, the term "urinary incontinence" includes disorders in which urination occurs when not desired, such as stress or urge incontinence. Other bladder dysfunctions may include disorders such as non-obstructive urinary retention.

One type of therapy for treating bladder dysfunction includes delivery of continuous electrical stimulation to a target tissue site within a patient to cause a therapeutic effect during delivery of the electrical stimulation. For example, delivery of electrical stimulation from an IMD to a target therapy site, e.g., a tissue site that delivers stimulation to modulate activity of a tibial nerve, spinal nerve (e.g., a sacral nerve), a pudendal nerve, dorsal genital nerve, an inferior rectal nerve, a perineal nerve, or branches of any of the aforementioned nerves, may provide an immediate therapeutic effect for bladder dysfunction, such as a desired reduction in frequency of bladder contractions. In some cases, electrical stimulation of the tibial nerve may modulate afferent nerve activities to restore urinary function during the electrical stimulation. However, continuous electrical stimulation (which may include pulsed stimulation) or other types of neurostimulation (e.g., drug delivery therapy) may provide neurostimulation during unnecessary phases of a physiological cycle that may cause undesirable side effects, accommodation, less focused therapy, and increased energy usage by the IMD delivering therapy.

In contrast to this type of continuous neurostimulation therapy, the example devices, systems, and techniques described in this disclosure are directed to managing delivery of neurostimulation therapy in a non-continuous manner which may include on-cycles and off-cycles. For example, an IMD may deliver neurostimulation therapy for a specified period of time followed by a specified period of time when the IMD does not deliver neurostimulation (e.g., withholds delivery of neurostimulation). During periods when the IMD is not delivering neurostimulation, the IMD may enter a hibernation mode to further reduce an amount of power or current draw on a battery powering the IMD. As described herein, a period during which stimulation is delivered (an on-cycle) may include on and off periods (e.g., a duty cycle or bursts of pulses) where even the short inter-pulse durations of time when pulses are not delivered are still considered part of the delivery of stimulation. In some examples, IMD 16 may not enter the hibernation mode during the short inter-pulse durations of time of an on-cycle when pulses are not delivered. For example, the short inter-pulse duration of time when pulses are not delivered during an on-cycle is still considered part of delivery of stimulation, but during an off-cycle, IMD 16 may be considered as not delivering stimulation.

The disclosure includes discussion of various examples, aspects, and features. Unless otherwise stated, the various examples, aspects, and features are contemplated as being used together in different combinations. For ease of discussion and as a practical matter, each possible combination of features is not expressly recited.

In some examples, a system may be configured to provide stimulation at a neural target located at a distant site from the end organ that is being affected. For example, stimulation sites may be located a relatively large distance from the bladder or bowel, such as a tibial nerve. The system may include of multiple devices (e.g., implantable sensors and implantable stimulation devices) with wireless communication circuitry that allows for wireless communication of information between the devices, which may provide of sensing or therapeutic stimulation. For example, the wireless circuitry may be designed to communicate using Near Field Communication, Bluetooth^{®}, or other wireless protocols.

For ease of discussion, various examples are discussed in connection with bladder function. It is to be recognized that bladder function is but one possible application. Various aspects of the present disclosure may also be used in connection with urinary, bowel, and general pelvic floor dysfunction. For the sake of brevity, each type of dysfunction is not repeated for each feature or example discussed herein.

As mentioned above, constant or continuous stimulation may result in undesirable side effects, accommodation, less focused therapy, and increased energy usage by the medical device delivering therapy. Therefore, stimulation may be cycled on and off. When stimulation is cycled off, the neurostimulation device may enter a hibernation mode in which very little current is drawn from the battery powering the neurostimulation device. This may translate into longer recharge intervals and/or longer replacement intervals.

A device, such as an IMD may implement the techniques described in this disclosure and deliver stimulation therapy to at least one nerve (e.g., tibial nerve, a sacral nerve, a spinal nerve, or a pelvic floor nerve) to modulate activity of the nerve via at least one electrode electrically connected to the IMD. The electrical stimulation may be configured to modulate contraction of a detrusor muscle of the patient to cause a decrease in frequency of bladder contractions (to reduce incontinence) or an increase in the frequency of bladder contractions (to promote voiding). Reduction in frequency of bladder contractions may reduce urgency of voiding and may reduce urgency and/or urinary incontinence, and thereby at least partially alleviate bladder dysfunction.

The neurostimulation described herein may be targeted to manage bladder dysfunction, such as an overactive bladder, urgency, urinary incontinence, or even non-obstructive urinary retention. For example, the stimulation may be delivered to target tissue sites normally used to alleviate these types of dysfunctions. Although the techniques are primarily described in this disclosure for managing bladder dysfunction, the techniques may also be applied to manage other pelvic floor disorders or disorders relating to other organs, tissues or nerves of the patient. For example, the devices, systems, and techniques described in this disclosure alternatively or additionally may be utilized to manage sexual dysfunction, pelvic pain, fecal urgency or fecal incontinence. Example nerves that may be targeted for therapy include tibial nerves, sacral nerves, pudendal nerves, a dorsal nerve of the penis or clitoris, sural nerves, sciatic nerves, the inferior rectal nerve, and peroneal or perineal nerves. Example organ systems that may be treated for dysfunction may include the large and small bowel, stomach and/or intestines, liver, and spleen, which may be modulated by delivering neurostimulation directly to the organs, to one or nerves innervating the organ, and/or blood supplies reaching the organs. In other examples, therapy may target a spinal cord for pain relief. In other examples, therapy may target a brain for treatment of Parkinson's disease or seizure disorders.

Various examples are discussed relative to one or more stimulation devices. It is recognized that the stimulation devices may include features and functionality in addition to electrical stimulation. Many of these additional features are expressly discussed herein. A few example features include, but are not limited to, different types of sensing capabilities and different types of wireless communication capabilities. For ease of discussion, the present disclosure does not expressly recite every conceivable combination the additional features, such as by repeating every feature each time different examples and uses of the stimulation devices are discussed. Moreover, the techniques describer herein for controlling the delivery of power within a device may be used in any device that is powered by a battery.

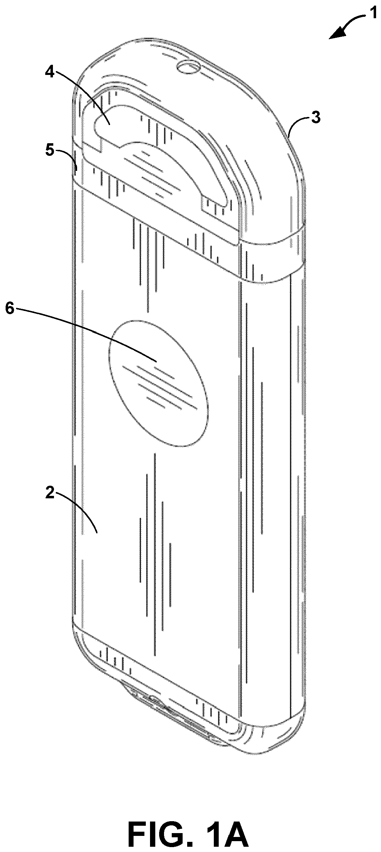

FIG 1A is a schematic diagram illustrating an example leadless neurostimulation device. Leadless neurostimulation device 1 includes a housing 2 containing components therein configured for delivering neurostimulation therapy, a header unit 3 that includes one or more primary electrodes 4, and a mounting plate 5 that couples housing 2 to header unit 3. Header unit 3 includes at least one primary electrode 4 that forms part of an exterior surface of header unit 3. Housing 2 includes a secondary electrode 6 that forms part of an exterior surface of housing 2 and is positioned on the same side of device 1 as primary electrode 4. In an alternate embodiment not depicted, primary electrode 4 and secondary electrode 6 may be arranged on opposite sides of device 1.

Primary electrode 4 and secondary electrode 6 operate in conjunction with one another to provide stimulation therapy to a target treatment site (e.g., a tibial nerve). Secondary electrode 6 may also be referred to as a case electrode, can electrode or reference electrode. In an example, primary electrode 4 may comprise a cathode and secondary electrode 6 may comprise an anode. In some examples, primary and secondary electrodes 4 and 6 may be characterized as a bipolar pair or system.

The terms "primary" and "secondary" are used to differentiate two or more electrodes that are configured to transmit an electrical signal therebetween. The terms are not used to imply a hierarchy among the electrodes, positive and negative terminal, a total number of electrodes, or a directionality by which a signal is transmitted between the electrodes.

Additional information relating to leadless neurostimulation device 1 may be found in U.S. Patent Publication 2022/0096845A1.

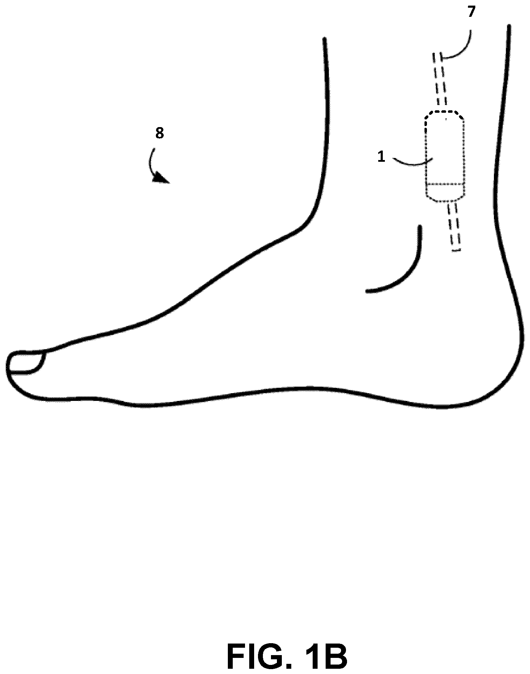

FIG. 1B is a conceptual diagram illustrating a leg having a leadless neurostimulation device implanted therein near a tibial nerve. In the example of FIG. 1B, leadless neurostimulation device 1 is implanted near tibial nerve 7 in leg 8 of a patient. For example, leadless neurostimulation device 1 may deliver neurostimulation to the patient to manage bladder dysfunction, such as overactive bladder, urgency, or urinary incontinence. Leadless neurostimulation device 1 may be configured to deliver neurostimulation to tibial nerve 7 in a cycled manner and place leadless neurostimulation device 1 in a hibernation mode when not actively delivering (e.g., withholding) neurostimulation (e.g., an off-cycle).

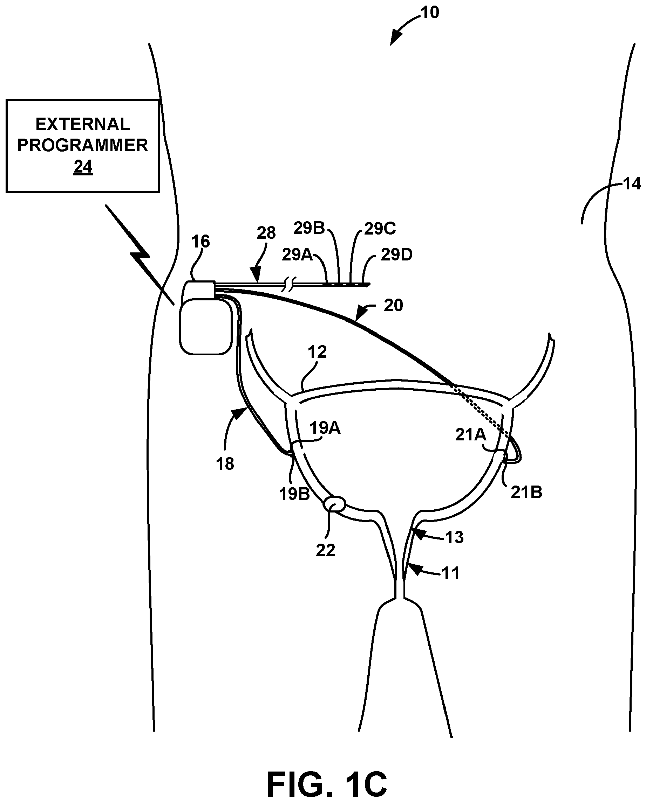

FIG. 1C is a conceptual diagram illustrating an example system 10 that manages delivery of neurostimulation to patient 14 to manage bladder dysfunction, such as overactive bladder, urgency, or urinary incontinence. As described above, system 10 may be configured to deliver neurostimulation to patient 14 in a cycled manner and place implantable medical device (IMD) 16 in a hibernation mode when not actively delivering (e.g., withholding) neurostimulation (e.g., an off-cycle).

As shown in the example of FIG. 1C, therapy system 10 includes IMD 16 (e.g., an example medical device), which is coupled to leads 18, 20, and 28 and sensor 22. System 10 also includes an external programmer 24, which is configured to communicate with IMD 16 via wireless communication. While not shown in FIGS. 1A-1B, external programmer 24 may be configured to communicate with leadless neurostimulation device 1.

IMD 16 generally operates as a therapy device that delivers electrical neurostimulation to, for example, a target tissue site proximate a sacral nerve, a tibial nerve, a spinal nerve, a pudendal nerve, dorsal genital nerve, an inferior rectal nerve, a perineal nerve, or other pelvic nerves, or branches of any of the aforementioned nerves. IMD 16 provides electrical stimulation to patient 14 by generating and delivering a programmable electrical stimulation signal (e.g., in the form of electrical pulses or an electrical waveform) to a target a therapy site near lead 28 and, more particularly, near electrodes 29A-29D (collectively referred to as "electrodes 29") disposed proximate to a distal end of lead 28.

IMD 16 may be surgically implanted in patient 14 at any suitable location within patient 14, such as near the pelvis. In some examples, IMD 16 may be implanted in a subcutaneous location in the side of the lower abdomen or the side of the lower back or upper buttocks. IMD 16 has a biocompatible housing, which may be formed from titanium, stainless steel, a liquid crystal polymer, or the like. The proximal ends of leads 18, 20, and 28 are both electrically and mechanically coupled to IMD 16 either directly or indirectly, e.g., via respective lead extensions. Electrical conductors disposed within the lead bodies of leads 18, 20, and 28 electrically connect sense electrodes (e.g., electrodes 19A, 19B, 21A, and 21B) and stimulation electrodes, such as electrodes 29, to sensing circuitry and a stimulation delivery circuitry (e.g., a stimulation generator) within IMD 16. In the example of FIG. 1C, leads 18 and 20 carry electrodes 19A, 19B (collective referred to as "electrodes 19") and electrodes 21A, 21B (collectively referred to as "electrodes 21"), respectively. As described in further detail below, electrodes 19 and 21 may be positioned for sensing an impedance of bladder 12, which may increase as the volume of urine within bladder 12 increases. In some examples, system 10 may include electrodes (such as electrodes 19 and 21), a strain gauge, one or more accelerometers, ultrasound sensors, optical sensors, or any other sensor capable of detecting contractions of bladder 12, pressure or volume of bladder 12, or any other indication of the fill cycle of bladder 12 and/or possible bladder dysfunctional states.

In other examples, system 10 may use sensors other than electrodes 19 and 21 for sensing bladder volume, or not use any sensors at all. For example, external programmer 24 may receive user input identifying a voiding event, perceived level of fullness, or the like. The user input may be in the form of a voiding journal analyzed by external programmer 24 or IMD 16 or individual user inputs associated with respective voiding events, leakage, or any other event related to a phase of the physiological cycle. External programmer 24 and/or IMD 16 may use this user input to generate estimated fill cycles and determine when to deliver stimulation and exit hibernation mode and when to withhold stimulation and enter hibernation mode. The user input may be in addition to or instead of sensors such as electrodes 19A and 21A for detecting a physiological marker.

One or more medical leads, e.g., leads 18, 20, and 28, may be connected to IMD 16 and surgically or percutaneously tunneled to place one or more electrodes carried by a distal end of the respective lead at a desired nerve or muscle site, e.g., one of the previously listed target therapy sites such as a tissue site proximate a sacral, tibial, spinal, or pudendal nerve. For example, lead 28 may be positioned such that electrodes 29 deliver electrical stimulation to a sacral, tibial, sacral, or pudendal nerve to reduce a frequency and/or magnitude of contractions of bladder 12. Additional electrodes of lead 28 and/or electrodes of another lead may provide additional stimulation therapy to other nerves or tissues as well. In FIG. 1C, leads 18 and 20 are placed proximate to an exterior surface of the wall of bladder 12 at first and second locations, respectively. In other examples of therapy system 10, IMD 16 may be coupled to more than one lead that includes electrodes for delivery of electrical stimulation to different stimulation sites within patient 14, e.g., to target different nerves.

In the example shown in FIG. 1C, leads 18, 20, 28 are cylindrical. Electrodes 19, 20, 29 of leads 18, 20, 28, respectively, may be ring electrodes, segmented electrodes, partial ring electrodes or any suitable electrode configuration. Segmented and partial ring electrodes each extend along an arc less than 360 degrees (e.g., 90-120 degrees) around the outer perimeter of the respective lead 18, 20, 28. In some examples, segmented electrodes 29 of lead 28 may be useful for targeting different fibers of the same or different nerves to generate different physiological effects (e.g., therapeutic effects). In examples, one or more of leads 18, 20, 28 may be, at least in part, paddle-shaped (e.g., a "paddle" lead), and may include an array of electrodes on a common surface, which may or may not be substantially flat.

In some examples, one or more of electrodes 19, 20, 29 may be cuff electrodes that are configured to extend at least partially around a nerve (e.g., extend axially around an outer surface of a nerve). Delivering electrical stimulation via one or more cuff electrodes and/or segmented electrodes may help achieve a more uniform electrical field or activation field distribution relative to the nerve, which may help minimize discomfort to patient 14 that results from the delivery of electrical stimulation. An electrical field may define the volume of tissue that is affected when the electrodes 19, 20, 29 are activated. An activation field represents the neurons that will be activated by the electrical field in the neural tissue proximate to the activated electrodes.

The illustrated numbers and configurations of leads 18, 20, and 28 and electrodes carried by leads 18, 20, and 28 are merely exemplary. Other configurations, e.g., numbers and positions of leads and electrodes are also contemplated. For example, in other implementations, IMD 16 may be coupled to additional leads or lead segments having one or more electrodes positioned at different locations proximate the spinal cord or in the pelvic region of patient 14. The additional leads may be used for delivering different stimulation therapies or other electrical stimulations to respective stimulation sites within patient 14 or for monitoring at least one physiological marker of patient 14.

In accordance with some examples of the disclosure, IMD 16 delivers electrical stimulation based on a stimulation program to at least one of a sacral nerve, tibial nerve, spinal nerve, a pudendal nerve, dorsal genital nerve, an inferior rectal nerve, or a perineal nerve to provide a therapeutic effect that reduces or eliminates a dysfunctional state such as overactive bladder. The desired therapeutic effect may be an inhibitory physiological response related to voiding of patient 14, such as a reduction in bladder contraction frequency by a desired level or degree (e.g., percentage).

The stimulation program may define various parameters of the stimulation waveform and electrode configuration which result in a predetermined stimulation intensity being delivered to the targeted nerve or tissue. In some examples, the stimulation program defines parameters for at least one of a current or voltage amplitude of the stimulation signal, a frequency or pulse rate of the stimulation, the shape of the stimulation waveform, a duty cycle of the stimulation, a pulse width of the stimulation, and/or the combination of electrodes 29 and respective polarities of the subset of electrodes 29 used to deliver the stimulation. Together, these stimulation parameter values may be used to define the stimulation intensity (also referred to herein as a stimulation intensity level). In some examples, if stimulation pulses are delivered in bursts, a burst duty cycle also may contribute to stimulation intensity. Also, independent of intensity, a particular pulse width and/or pulse rate may be selected from a range suitable for causing the desired therapeutic effect after stimulation is terminated and, optionally, during stimulation. In addition, as described herein, a period during which stimulation is delivered may include on and off periods (e.g., a duty cycle or bursts of pulses) where even the short inter-pulse durations of time when pulses are not delivered are still considered part of the delivery of stimulation. In some examples, IMD 16 may not enter the hibernation mode during the short inter-pulse durations of time when pulses are not delivered. For example, the short inter-pulse duration of time when pulses are not delivered during an on-cycle is still considered part of delivery of stimulation, but during an off-cycle, IMD 16 may be considered as not delivering stimulation.

The stimulation programs may also define a period during which IMD 16 delivers stimulation (e.g., on-cycle) and a period during which IMD 16 withholds stimulation delivery (e.g., off-cycle). The period during which IMD 16 withholds stimulation delivery is a period in which no stimulation program is active for IMD 16 (e.g., IMD 16 is not tracking pulse durations or inter-pulse durations that occur as part of the electrical stimulation delivery scheme). During such times, IMD 16 may enter the hibernation mode. Typically, a period during which IMD 16 withholds neurostimulation is on the order of weeks, days, minutes or hours, not tenths of a second or several seconds.

These periods (e.g., time period for the on-cycle and time period for the off-cycle) may be programmed to be absolute or linked to triggers, such as sensor signals or telemetry circuitry signals. In some examples, IMD 16 may be configured to deliver different types of stimulation therapy at different times during a physiological cycle of patient 14. For example, IMD 16 may deliver stimulation configured to reduce or eliminate bladder contractions to promote urine retention and/or increased bladder capacity and then deliver stimulation configured to promote urination (e.g., increased frequency or magnitude of bladder contractions) for a user requested voiding event or once a voiding event is detected to have begun.

In some examples, the hibernation mode includes a plurality of hibernation periods in a sequence, with brief periods of time out of hibernation mode. For example, IMD 16 may come out of hibernation mode to run diagnostic tests or advertise for telemetry, before reentering hibernation mode if there is not an issue with the results of the diagnostic tests or a request to connect to an external device. In some examples, the hibernation time can be varied by algorithms that monitor therapy efficacy (e.g., using implanted or external sensors) and adjust the therapy stimulation dose.

System 10 may also include an external programmer 24, as shown in FIG. 1C. External programmer 24 may be a clinician programmer or patient programmer. In some examples, external programmer 24 may be a wearable communication device, with a therapy request input integrated into a key fob or a wristwatch, handheld computing device, smart phone, computer workstation, or networked computing device. External programmer 24 may include a user interface that is configured to receive input from a user (e.g., patient 14, a patient caretaker, or a clinician). In some examples, the user interface includes, for example, a keypad and a display, which may for example, be a liquid crystal display (LCD) or light emitting diode (LED) display. The keypad may take the form of an alphanumeric keypad or a reduced set of keys associated with particular functions. External programmer 24 may additionally or alternatively include a peripheral pointing device, such as a mouse, via which a user may interact with the user interface. In some examples, a display of external programmer 24 may include a touch screen display, and a user may interact with external programmer 24 via the display. It should be noted that the user may also interact with external programmer 24 and/or IMD 16 remotely via a networked computing device. In some examples, external programmer 24 may be configured to interoperate with leadless neurostimulation device 1 of FIGS. 1A-1B.

A user, such as a physician, technician, surgeon, electrophysiologist, or other clinician, may also interact with external programmer 24 or another separate programmer (not shown), such as a clinician programmer, to communicate with IMD 16. Such a user may interact with a programmer to retrieve physiological or diagnostic information from IMD 16. The user may also interact with a programmer to program IMD 16, e.g., select values for the stimulation parameter values with which IMD 16 generates and delivers stimulation and/or the other operational parameters of IMD 16, such as magnitudes of stimulation energy, user requested periods for stimulation or periods to prevent stimulation, or any other such user customization of therapy. As discussed herein, the user may also provide input to external programmer 24 indicative of physiological events such as bladder fill level perception and void events.

For example, the user may use a programmer to retrieve information from IMD 16 regarding the contraction frequency of bladder 12 and/or voiding events. As another example, the user may use a programmer to retrieve information from IMD 16 regarding the performance or integrity of IMD 16 or other components of system 10, such as leads 18, 20, and 28, or a power source of IMD 16. In some examples, this information may be presented to the user as an alert if a system condition that may affect the efficacy of therapy is detected.

Patient 14 may, for example, use a keypad or touch screen of external programmer 24 to request IMD 16 to deliver or terminate the electrical stimulation, such as when patient 14 senses that a leaking episode may be imminent or when an upcoming void may benefit from terminating therapy that promotes urine retention. In this way, patient 14 may use external programmer 24 to provide a therapy request to control the delivery of the electrical stimulation "on demand," e.g., when patient 14 deems the stimulation therapy desirable. In the case where patient 14 uses external programmer 24 to request IMD 16 to deliver therapy, IMD 16 may exit the hibernation mode in order to deliver the electrical stimulation. In some examples, patient 14 may use external programmer 24 to request IMD 16 to terminate electrical stimulation. In the case where patient 14 uses external programmer 24 to terminate electrical stimulation, IMD 16 may enter hibernation mode. Patient 14 may also use external programmer 24 to provide other information to IMD 16, such as information indicative of a phase of a physiological cycle, such as the occurrence of a voiding event.

External programmer 24 may provide a notification to patient 14 when the electrical stimulation is being delivered or notify patient 14 of the prospective termination of the electrical stimulation. In addition, notification of termination may be helpful so that patient 14 knows that a voiding event may be more probable and/or the end of the fill cycle is nearing such that the bladder should be emptied (e.g., patient 14 should visit a restroom). In such examples, external programmer 24 may display a visible message, emit an audible alert signal or provide a somatosensory alert (e.g., by causing a housing of external programmer 24 to vibrate). In other examples, the notification may indicate when therapy is available (e.g., a countdown in minutes, or indication that therapy is ready) during the physiological cycle. In this manner, external programmer 24 may wait for input from patient 14 prior to terminating the electrical stimulation that reduces bladder contraction or otherwise promotes urine retention. Patient 14 may enter input that either confirms termination of the electrical stimulation so that the therapy stops for voiding purposes, confirms that the system should maintain therapy delivery until patient 14 may void, and/or confirms that patient 14 is ready for another different stimulation therapy that promotes voiding during the voiding event.

In the event that no input is received within a particular range of time when a voiding event is predicted, external programmer 24 may wirelessly transmit a signal that indicates the absence of patient input to IMD 16. IMD 16 may then elect to continue stimulation until the patient input is received, or terminate stimulation to avoid tissue damage, based on the programming of IMD 16.

IMD 16 and external programmer 24 may communicate via wireless communication using any techniques known in the art. Examples of communication techniques may include, for example, low frequency, radiofrequency (RF) telemetry, or inductive coupling, but other techniques are also contemplated. In some examples, external programmer 24 may include a programming lead that may be placed proximate to the body of patient 14 near the IMD 16 implant site in order to improve the quality or security of communication between IMD 16 and external programmer 24.

In one example described herein, a device (e.g., IMD 16) includes a battery configured to provide power to the device. The device includes power domain firmware configured to determine when to open or keep closed a battery switch based on one or more of a plurality of requests which the power domain firmware may receive from a plurality of firmware modules. When the battery switch is open, the device is in the hibernation mode described above and power is not supplied to many components of the device, whereas when the battery switch is closed, power is supplied to all components of the device requiring power. The device includes the battery switch and the plurality of firmware modules. In examples where the device includes stimulation circuitry, the stimulation circuitry is not powered by the battery when the medical device is in the hibernation mode (e.g., the battery switch is open).

Unlike other techniques of entering or exiting a hibernation mode, an implantable neurostimulator, such as leadless neurostimulation device 1 or IMD 16, may include a plurality of firmware modules executing on processing circuitry and a power domain firmware module executing on power domain circuitry. Each of the plurality of firmware modules are configured to perform respective functions of the device (e.g., therapy delivery, telemetry, etc.). At least two of the plurality of firmware modules are configured to determine whether power is needed for a corresponding hardware component (e.g., therapy delivery circuitry, telemetry circuitry, etc.) or to perform the respective function during a respective time period. The at least two of the plurality of firmware modules are also configured to generate and transmit one or more respective requests based on the determination of whether power is needed for the corresponding hardware component or to perform the respective function during the respective time period. The power domain firmware module is configured to receive the one or more respective requests. and determine whether to open the battery switch or to keep the battery switch closed in response to the one or more respective requests. The power domain firmware module is also configured to control the battery switch to open or keep closed in response to the determination.

As described above, there may be different physiological markers. As one example, the magnitude of the fill level may be a physiological marker for the bladder fill cycle. In one example, system 10 may detect the magnitude of the fill level by detecting a pressure level of bladder 12 (e.g., via sensor 22). For example, one or more pressure or stretch sensors may be attached to the exterior of bladder 12 or implanted within the bladder. As another example, system 10 may detect the magnitude of the fill level by detecting an impedance level of bladder 12, such as by monitoring the impedance between electrodes 19 and 21 of FIG. 1C.

IMD 16 may detect a contraction of bladder 12 using any suitable technique, such as based on a sensed one or more physiological parameters that may be a physiological marker for the physiological cycle. In one example, a physiological marker is an impedance of bladder 12. In the example shown in FIG 1, IMD 16 may determine impedance of bladder 12 using a four-wire (or Kelvin) measurement technique. In other examples, IMD 16 may measure bladder impedance using a two-wire sensing arrangement. In either case, IMD 16 may transmit an electrical measurement signal, such as a current, through bladder 12 via leads 18 and 20, and determine impedance of bladder 12 based on the transmitted electrical signal. Such an impedance measurement may be utilized to determine a fullness of bladder 12, or the like.

In the example four-wire arrangement shown in FIG. 1C, electrodes 19A and 21A and electrodes 19B and 21B, may be located substantially opposite each other relative to the center of bladder 12. For example, electrodes 19A and 21A may be placed on opposing sides of bladder 12, either anterior and posterior or left and right. In FIG. 1C, electrodes 19 and 21 are shown placed proximate to an exterior surface of the wall of bladder 12. In some examples, electrodes 19 and 21 may be sutured or otherwise affixed to the bladder wall. In other examples, electrodes 19 and 21 may be implanted within the bladder wall. To measure the impedance of bladder 12, IMD 16 may source an electrical signal, such as current, to electrode 19A via lead 18, while electrode 21A via lead 20 sinks the electrical signal. IMD 16 may then determine the voltage between electrode 19B and electrode 21B via leads 18 and 20, respectively. IMD 16 determines the impedance of bladder 12 using a known value of the electrical signal sourced the determined voltage.

In other examples, electrodes 19 and 21 may be used to detect an EMG of the detrusor muscle. This EMG may be used to determine the frequency of bladder contractions and the physiological marker of patient 14. The EMG may also be used to detect the strength of the bladder contractions in some examples. As an alternative, or in addition, to an EMG, a strain gauge or other device may be used to detect the status of bladder 12, e.g., by sensing forces indicative of bladder contractions.

In the example of FIG. 1C, IMD 16 also includes a sensor 22 for detecting changes in the contraction of bladder 12. Sensor 22 may include, for example, a pressure sensor for detecting changes in bladder pressure, electrodes for sensing tibial, pudendal, or sacral afferent nerve signals, electrodes for sensing urinary sphincter EMG signals (or anal sphincter EMG signals in examples in which system 10 provides therapy to manage fecal urgency or fecal incontinence), or any combination thereof. In examples in which sensor 22 is a pressure sensor or a stretch sensor, sensor 22 may be a remote sensor that wirelessly transmits signals to IMD 16 or may be carried on one of leads 18, 20, or 28 or an additional lead coupled to IMD 16. In some examples, IMD 16 may determine whether a contraction frequency of bladder 12 has occurred based on a pressure signal generated by sensor 22.

In examples in which sensor 22 includes one or more electrodes for sensing afferent nerve signals, the sense electrodes may be carried on one of leads 18, 20, or 28 or an additional lead coupled to IMD 16. In examples in which sensor 22 includes one or more sense electrodes for generating a urinary sphincter EMG, the sense electrodes may be carried on one of leads 18, 20, or 28 or additional leads coupled to IMD 16. In any case, in some examples, IMD 16 may control the timing of the delivery of the electrical stimulation based on input received from sensor 22.

Sensor 22 may comprise a patient motion sensor, such as an accelerometer, that generates a signal indicative of patient activity level or posture state. In some examples, IMD 16 may terminate or resume the delivery of the electrical stimulation to patient 14 upon detecting a patient activity level falling below or exceeding a particular threshold based on the signal from the motion sensor. For example, if the patient activity level that is greater than or equal to a threshold (which may be stored in a memory of IMD 16) may indicate that there is an increase in the probability that an involuntary voiding event will occur, and, therefore, system 10 should exit the hibernation mode and begin delivering electrical stimulation. In other examples, IMD 16 may use sensor 22 to identify posture states known to require the desired therapeutic effect. For example, patient 14 may be more prone to an involuntary voiding event when patient 14 is in an upright posture state compared to a lying down posture state. In any event, electrodes 19 and 21 and sensor 22 may be configured to detect voiding events and/or the magnitude of a fill level of bladder 12 during the fill cycle.

As discussed above, system 10 may monitor the fill cycle of bladder 12 by detecting subsequent voiding events over time. In some examples, system 10 may detect voiding events by receiving an indication of a user input (e.g., via external programmer 24) representative of an occurrence of a voiding event. In other words, external programmer 24 may receive input from the user identifying that a voiding event occurred, the beginning of a voiding event, and/or the end of the voiding event. In other examples, system 10 may automatically detect voiding events without receiving user input via external programmer 24. System 10 may instead detect voiding events by detecting at least one of a pressure of the bladder, a flow of urine from the bladder, a wetness of an article external to patient 14, a volume of the bladder, an electromyogram (EMG) signal, a nerve recording, a posture change, a physical location of patient 14 within a structure such as a house or care facility, or a toilet use event. Some sensors external to patient 14 may communicate with external programmer 24 and/or IMD 16 to provide this information indicative of likely voiding events. For example, wetness may be detected by a moisture sensor (e.g., electrical impedance or chemical sensor) embedded in an undergarment worn by patient 14 and transmitted to IMD 16 or external programmer 24. Similarly, a toilet may include a presence sensor that detects when patient 14 is using the toilet (e.g., an infrared sensor, thermal sensor, or pressure senor) and transmits a signal indicating the presence of patient 14 to IMD 16 or external programmer 24. In this manner, non-invasively obtained data may provide information indicative of voiding events without implanted sensors, and such information may be used to determine when to enter or exit the hibernation mode.

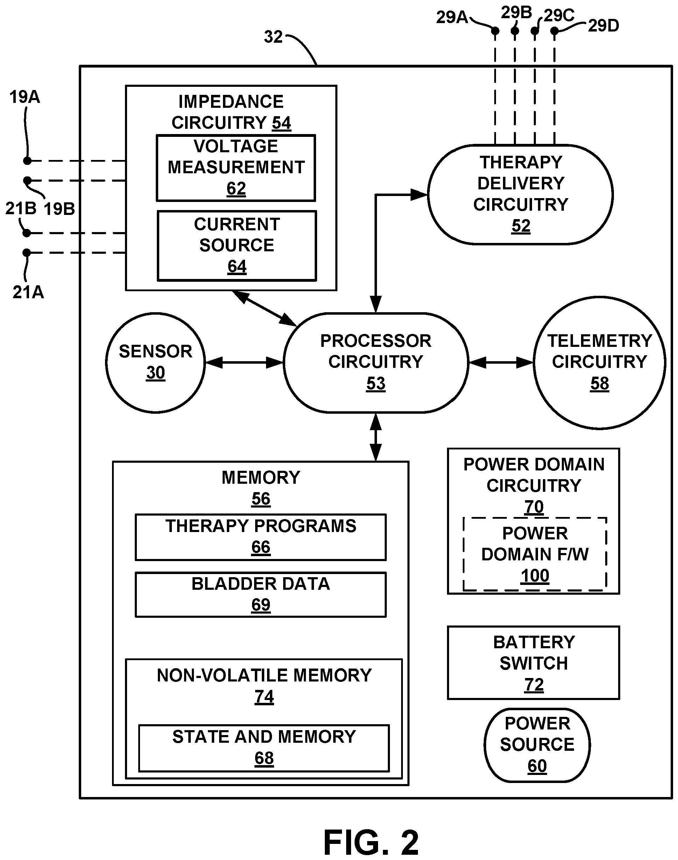

FIG. 2 is a block diagram illustrating an example configuration of an IMD. As shown in FIG. 2, IMD 32, which may be an example of leadless neurostimulation device 1 or IMD 16, includes sensor 30 processor circuitry 53, therapy delivery circuitry 52, impedance circuitry 54, memory 56, telemetry circuitry 58, power domain circuitry 70, battery switch 72, and power source 60. Sensor 30 may, in some examples, be similar to sensor 22 of FIG. 1C. In other examples, IMD 32 may include a greater number of, fewer number of, or different components. For example, in the case where IMD 32 represents leadless neurostimulation device 1, IMD 32 does not include leads, but rather electrodes are disposed on the surface of IMD 32.

In general, IMD 32 may comprise any suitable arrangement of hardware, alone or in combination with software and/or firmware, to perform the techniques attributed to IMD 32 and components of IMD 32. In various examples, processor circuitry 53 of IMD 32 may include one or more processors, such as one or more microprocessors, digital signal processors (DSPs), application specific integrated circuits (ASICs), field programmable gate arrays (FPGAs), or any other equivalent integrated or discrete logic circuitry, as well as any combinations of such components. IMD 32 also, in various examples, may include a memory 56, such as random-access memory (RAM), synchronous RAM (SRAM), ferroelectric RAM (FRAM), non-volatile memory, read only memory (ROM), programmable read only memory (PROM), erasable programmable read only memory (EPROM), electronically erasable programmable read only memory (EEPROM), flash memory, comprising executable instructions for causing the one or more processors to perform the actions attributed to them. In some examples, memory 56 includes non-volatile memory 74 which may be used to store values, such as state and memory information 68, used by components, such as firmware components, that may be unpowered when battery switch 72 is open in order to preserve such values.

Although processor circuitry 53, therapy delivery circuitry 52, impedance circuitry 54, and telemetry circuitry 58 are described as separate circuitry, in some examples, processor circuitry 53, therapy delivery circuitry 52, impedance circuitry 54, and telemetry circuitry 58 are functionally integrated. In some examples, processor circuitry 53, therapy delivery circuitry 52, impedance circuitry 54, and telemetry circuitry 58 correspond to individual hardware units, such as microprocessors, ASICs, DSPs, FPGAs, or other hardware units. In further examples, any of processor circuitry 53, therapy delivery circuitry 52, impedance circuitry 54, and telemetry circuitry 58 may correspond to multiple individual hardware units such as microprocessors, ASICs, DSPs, FPGAs, or other hardware units.

Memory 56 stores therapy programs 66 that specify stimulation parameter values and electrode combinations for the electrical stimulation provided by IMD 32. Therapy programs 66 may also store information regarding determining and using physiological parameters, information regarding physiological cycles and/or dysfunctional states, or any other information required by IMD 32 to deliver stimulation therapy. In some examples, the stimulation therapy is based on one or more physiological parameters of patient 14. In some examples, memory 56 also stores bladder data 69, which processor circuitry 53 may use for controlling the timing of the delivery of the electrical stimulation (e.g., phases of physiological cycles that define when to deliver and withhold stimulation). For example, bladder data 69 may include threshold values or baseline values for at least one of bladder impedance, bladder pressure, afferent nerve signals, bladder contraction frequency, or external urinary sphincter EMG templates for use as physiological markers for an associated physiological cycle. Bladder data 69 may also include timing information and physiological markers associated with physiological events, such as a voiding event.

Memory 56 may also store state and memory information 68. In some examples, non-volatile memory 74 may store state and memory information 68 so that state and memory information 68 is stored even when battery switch 72 is open. For example, when IMD 32 is about to enter the hibernation mode, processor circuitry 53, power domain circuitry 70, or power domain firmware (not shown in FIG. 2), may store certain state and memory information in state and memory information 68. Battery switch 72 may then open. State and memory information 68 may then be accessed by various components of IMD 32, such as firmware, processor circuitry 53, or the like, when IMD 32 is exiting the hibernation mode, which may be used to restore IMD 32 to a normal operative state. State and memory information 68 may include time information indicative of when the hibernation mode is being entered, a desired exit time from hibernation mode, length of time of last therapy delivery, or the like. State and memory information 68 may include diagnostic information, such as mirrors and logs, and/or use information. State and memory information 68 may also include information regarding sensor acquisition and therapy sequences. State and memory information 68 may include algorithm states and data for assessments that are underway, such as bladder filling calculations or therapy titration calculations. State and memory information 68 may also include diagnostic data for the device state, such as battery voltage, electrode impedances, and/or telemetry logs. It should be noted that when battery switch 72 is closed, state and memory information 68 may be stored in other types of memory of memory 56, such as RAM, SRAM, FRAM, etc., rather than in non-volatile memory 74.

In some examples, power domain circuitry 70 may remain powered when battery switch 72 is open. In some examples, power domain circuitry 70 may close battery switch 72 to re-power other components of IMD 32. For example, power domain circuitry 70 may close battery switch 72 in response to detecting incoming telemetry, detecting recharge energy, or after a defined time period (e.g., a time period received from power domain firmware). In some examples, each of the other components of IMD 32 is powered down when battery switch 72 is open, except for a real-time clock (not shown) and circuitry or firmware that may be configured to detect an incoming telemetry transmission and/or recharge energy used to recharge the battery.

Information related to sensed bladder contractions, bladder impedance and/or posture of patient 14 may be stored in bladder data 69. Bladder data 69 may be retrieved by a user, and/or used by processor circuitry 53 for adjustment of stimulation parameters (e.g., amplitude, pulse width, and pulse rate). In some examples, memory 56 includes separate memories for storing instructions, electrical signal information, therapy programs 66, state and memory information 68, and bladder data 69. In some examples, processor circuitry 53 selects new stimulation parameters for a therapy program 66 or new stimulation program from therapy programs 66 to use in the delivery of the electrical stimulation based on patient input or sensor signals. In some examples, processor circuitry 53 may use bladder data 69 to determine efficacy of a therapy program and may adjust periods of stimulation and periods of withholding of stimulation and the associated operational mode and hibernation mode based on the determined efficacy of the therapy program.

Generally, therapy delivery circuitry 52 generates and delivers electrical stimulation under the control of processor circuitry 53. As used herein, controlling the delivery of electrical stimulation may also include controlling the termination of stimulation to achieve the different stimulation and non-stimulation phases. In some examples, processor circuitry 53 controls therapy delivery circuitry 52 by accessing memory 56 to selectively access and load at least one of therapy programs 66 to therapy delivery circuitry 52. For example, in operation, processor circuitry 53 may access memory 56 to load one of therapy programs 66 to therapy delivery circuitry 52. In other examples, therapy delivery circuitry 52 may access memory 56 and load one of the therapy programs 66.

By way of example, processor circuitry 53 may access memory 56 to load one of therapy programs 66 to therapy delivery circuitry 52 for delivering the electrical stimulation to patient 14. A clinician or patient 14 may select a particular one of therapy programs 66 from a list using a programming device, such as external programmer 24 or a clinician programmer. Processor circuitry 53 may receive the selection via telemetry circuitry 58. Therapy delivery circuitry 52 delivers the electrical stimulation to patient 14 according to the selected program for an extended period of time, such as minutes, hours, days, weeks, or until patient 14 or a clinician manually stops or changes the program.

Therapy delivery circuitry 52 delivers electrical stimulation according to stimulation parameters. In some examples, therapy delivery circuitry 52 delivers electrical stimulation in the form of electrical pulses. In such examples, relevant stimulation parameters may include a voltage amplitude, a current amplitude, a pulse rate, a pulse width, a duty cycle, or the combination of electrodes 29 that therapy delivery circuitry 52 uses to deliver the stimulation signal. In other examples, therapy delivery circuitry 52 delivers electrical stimulation in the form of continuous waveforms. In such examples, relevant stimulation parameters may include a voltage or current amplitude, a frequency, a shape of the stimulation signal, a duty cycle of the stimulation signal, or the combination of electrodes 29 therapy delivery circuitry 52 uses to deliver the stimulation signal.

In some examples, the stimulation parameters for the therapy programs 66 may be selected to relax bladder 12, e.g., to reduce a frequency of contractions of bladder 12, after termination of the electrical stimulation. An example range of stimulation parameters for the electrical stimulation that are likely to be effective in treating bladder dysfunction, e.g., upon application to the tibial, spinal, sacral, pudendal, dorsal genital, inferior rectal, or perineal nerves, are as follows:
1. Frequency or pulse rate: between about 0.5 Hz and about 500 Hz, such as between about 1 Hz and about 250 Hz, between about 1 Hz and about 20 Hz, or about 10 Hz.
2. Amplitude: between about 0.1 volts and about 50 volts, such as between about 0.5 volts and about 20 volts, or between about 1 volt and about 10 volts. Alternatively, the amplitude may be between about 0.1 milliamps (mA) and about 50 mA, such as between about 0.5 mA and about 20 mA, or between about 1 mA and about 10 mA.
3. Pulse Width: between about 10 microseconds (µs) and about 5000 µs, such as between about 100 µs and about 1000 µs, or between about 100 µs and about 200 µs.

When IMD 32 is monitoring the fill level of the bladder to determine the status of the bladder fill cycle, processor circuitry 53 may monitor impedance of bladder 12 for a predetermined duration of time to detect contractions of bladder 12, and determine the baseline contraction frequency of bladder 12 by determining a number of contractions of bladder 12 in the predetermined duration of time. In other examples, electrodes 19 or 21 may be used to detect an EMG of the detrusor muscle to identify bladder contraction frequencies. Alternatively, a strain gauge sensor signal output or other measure of bladder contraction change may be used to detect the physiological marker of bladder 12. Each of these alternative methods of monitoring the fill level and/or voiding event of bladder 12 may be used in some examples.

In the example illustrated in FIG. 2, impedance circuitry 54 includes voltage measurement circuitry 62 and current source 64, and may include an oscillator (not shown) or the like for producing an alternating signal. In some examples, as described above with respect to FIG. 1C, impedance circuitry 54 may use a four-wire, or Kelvin, arrangement. As an example, processor circuitry 53 may periodically control current source 64 to, for example, source an electrical current signal through electrode 19A and sink the electrical current signal through electrode 21A. In some examples, for collection of impedance measurements, current source 64 may deliver electrical current signals that do not deliver stimulation therapy to bladder 12, e.g., sub-threshold signals, due to, for example, the amplitudes or widths of such signals and/or the timing of delivery of such signals. Impedance circuitry 54 may also include a switching circuitry (not shown) for selectively coupling electrodes 19A, 19B, 21A, and 21B to current source 64 and voltage measurement circuitry 62. Voltage measurement circuitry 62 may measure the voltage between electrodes 19B and 21B. Voltage measurement circuitry 62 may include sample and hold circuitry or other suitable circuitry for measuring voltage amplitudes. Processor circuitry 53 determines an impedance value from the measure voltage values received from voltage measurement circuitry 62.

In other examples, processor circuitry 53 may monitor signals received from sensor 22 to detect contraction of bladder 12 and determine the baseline contraction frequency. In some examples, sensor 22 may be a pressure sensor for detecting changes in pressure of bladder 12, which processor circuitry 53 may correlate to contractions of bladder 12. Processor circuitry 53 may determine a pressure value based on signals received from sensor 22 and compare the determined pressure value to a threshold value stored in bladder data 69 to determine whether the signal is indicative of a contraction of bladder 12. In some implementations, processor circuitry 53 monitors pressure of bladder 12 to detect contractions of bladder 12 for a predetermined duration of time, and determines a contraction frequency of bladder 12 by calculating a number of contractions of bladder 12 in the predetermined time period.

In some examples, processor circuitry 53 may cause contraction frequency information to be stored as bladder data 69 in memory 56, and may utilize the changes to contraction frequency to track the fill level of the bladder fill cycle or otherwise track the phase of the fill cycle. In some implementations, processor circuitry 53 may, automatically or under control of a user, determine the contraction frequency over the fill cycle. Processor circuitry 53 may determine that an increase in contraction frequency indicates a later phase of the fill cycle. In some examples, processor circuitry 53 may track bladder contractions using EMG signals of patient 14. In some implementations, sensor 22 may include an EMG sensor, and processor circuitry 53 may generate an EMG from the received signals generated by sensor 22. Sensor 22 may be implanted proximate to a muscle which is active when bladder 12 is contracting, such as a detrusor muscle. Processor circuitry 53 may compare an EMG collected during the second time period to EMG templates stored as bladder data 69 (e.g., a short-term running average) to determine whether the contractions of bladder 12 are indicative of particular phases of the bladder fill cycle.

In other examples, sensor 22 may be a pressure sensor and processor circuitry 53 may monitor signals received from sensor 22 during at least a portion of the second time period to detect contraction of bladder 12. In some examples, processor circuitry 53 substantially continuously monitors pressure of bladder 12, at least during the second time periods, to detect contraction of bladder 12, and determines a contraction frequency of bladder 12 by determining a number of contractions of bladder 12 in a specified time period. Sensor 22 may also provide longer-term changes in pressure to track the bladder fill status (e.g., increased bladder volume may correspond to increased bladder pressure).

In the example of FIG. 2, therapy delivery circuitry 52 drives electrodes on a single lead 28. Specifically, therapy delivery circuitry 52 delivers electrical stimulation to tissue of patient 14 via selected electrodes 29A-29D carried by lead 28. A proximal end of lead 28 extends from the housing of IMD 32 and a distal end of lead 28 extends to a target therapy site, such as a tibial nerve, a spinal nerve (e.g., an S3 nerve), or a therapy site within the pelvic floor, such as tissue sites proximate a sacral nerve, a pudendal nerve, a dorsal genital nerve, an inferior rectal nerve, a perineal nerve, a hypogastric nerve, a urinary sphincter, or any combination thereof. In other examples, therapy delivery circuitry 52 may deliver electrical stimulation with electrodes on more than one lead and each of the leads may carry one or more electrodes. The leads may be configured as an axial lead with ring electrodes or segmented electrodes and/or paddle leads with electrode pads arranged in a two-dimensional array. The electrodes may operate in a bipolar or multi-polar configuration with other electrodes, or may operate in a unipolar configuration referenced to an electrode carried by the device housing or "can" of IMD 32.

As previously described, sensor 22 may comprise a pressure sensor configured to detect changes in bladder pressure, electrodes for sensing pudendal or sacral afferent nerve signals, or electrodes for sensing external urinary sphincter EMG signals (or anal sphincter signals in examples in which IMD 32 provides fecal urgency or fecal incontinence therapy), or any combination thereof. Additionally, or alternatively, sensor 22 may comprise a motion sensor, such as a two-axis accelerometer, three-axis accelerometer, one or more gyroscopes, pressure transducers, piezoelectric crystals, or other sensors that generate a signal that changes as patient activity level or posture state changes. Processor circuitry 53 may detect a physiological marker indicative of point during a bladder fill cycle. Sensor 22 may also be a motion sensor that is responsive to tapping (e.g., by patient 14) on skin superior to IMD 32. Processor circuitry 53 may be configured to log patient input using this tapping method (e.g., tapping may indicate that a voiding event is occurring). Alternatively, or in addition, processor circuitry 53 may control therapy circuitry 52 to deliver or terminate electrical stimulation delivery in response to the tapping or certain pattern of tapping.

In examples in which sensor 22 includes a motion sensor, processor circuitry 53 may determine a patient activity level or posture state based on a signal generated by sensor 22. This patient activity level may be, for example, sitting, exercising, working, running, walking, or any other activity of patient 14. For example, processor circuitry 53 may determine a patient activity level by sampling the signal from sensor 22 and determining a number of activity counts during a sample period, where each activity level of a plurality of activity levels is associated with respective activity counts. In one example, processor circuitry 53 compares the signal generated by sensor 22 to one or more amplitude thresholds stored within memory 56, and identifies each threshold crossing as an activity count. The physical activity may be indicative of a fill level, a voiding event, or any other physiological marker related to the bladder fill cycle.

In some examples, processor circuitry 53 may control therapy delivery circuitry 52 to deliver or terminate the electrical stimulation. Power domain circuitry 70 may control battery switch 72 to enter or exit the hibernation mode.

Telemetry circuitry 58 includes any suitable hardware, firmware, software or any combination thereof for communicating with another device, such as external programmer 24 (FIG. 1C). Under the control of processor circuitry 53, telemetry circuitry 58 may receive downlink telemetry, e.g., patient input, from and send uplink telemetry, e.g., an alert, to external programmer 24 with the aid of an antenna, which may be internal and/or external. Processor circuitry 53 may provide the data to be uplinked to external programmer 24 and the control signals for the telemetry circuit within telemetry circuitry 58, and receive data from telemetry circuitry 58.

Generally, processor circuitry 53 may control telemetry circuitry 58 to exchange information with external programmer 24 and/or another device external to IMD 32. Processor circuitry 53 may transmit operational information and receive stimulation programs or stimulation parameter adjustments via telemetry circuitry 58. Also, in some examples, IMD 32 may communicate with other implanted devices, such as stimulators, control devices, or sensors, via telemetry circuitry 58.

Power source 60 delivers operating power to the components of IMD 32. Power source 60 may include a battery and a power generation circuit to produce the operating power. In some examples, the battery may be rechargeable to allow extended operation. Recharging may be accomplished through proximal inductive interaction between an external charger and an inductive charging coil within IMD 32.

Power domain circuitry 70 (and/or power domain firmware not shown in FIG. 2) may be configured to remove power normally provided by power source 60 from various components of IMD 32. In some examples, power domain firmware 100, executing on processing circuitry, such as power domain circuitry 70, may be configured to determine whether IMD 32 should enter hibernation mode or not (e.g., open battery switch 72 or keep battery switch 72 closed), based on requests from a plurality of firmware modules (not shown in FIG. 2). For example, power domain circuitry 70 may be configured to receive a notification from power domain firmware that it is okay to open battery switch 72 and, in response to the notification, remove power from processor circuitry 53, therapy delivery circuitry 52, and/or impedance circuitry 54 by opening battery switch 72 and entering the hibernation mode. In some examples, power domain circuitry 70 may also be configured to restore power to such components upon exiting the hibernation mode. For example, power domain circuitry 70 may close battery switch 72 in response to detecting incoming telemetry, detecting recharge energy, or after a defined time period (e.g., a time period received from power domain firmware).

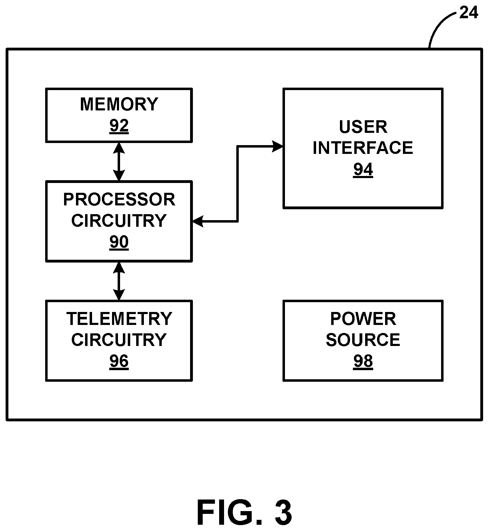

FIG. 3 is a block diagram illustrating an example configuration of an external programmer 24. While external programmer 24 may generally be described as a hand-held computing device, external programmer 24 may be a notebook computer, a smart phone, or a workstation, for example. As illustrated in FIG. 3, external programmer 24 may include a processor circuitry 90, memory 92, user interface 94, telemetry circuitry 96, and power source 98. Memory 92 may store program instructions that, when executed by processor circuitry 90, cause processor circuitry 90 and external programmer 24 to provide the functionality ascribed to external programmer 24 throughout this disclosure.

In general, external programmer 24 comprises any suitable arrangement of hardware, alone or in combination with software and/or firmware, to perform the techniques attributed to external programmer 24, and processor circuitry 90, user interface 94, and telemetry circuitry 96 of external programmer 24. In various examples, external programmer 24 may include one or more processors, such as one or more microprocessors, DSPs, ASICs, FPGAs, or any other equivalent integrated or discrete logic circuitry, as well as any combinations of such components. External programmer 24 also, in various examples, may include a memory 92, such as RAM, ROM, PROM, EPROM, EEPROM, flash memory, a hard disk, a CD-ROM, comprising executable instructions for causing the one or more processors to perform the actions attributed to them. Moreover, although processor circuitry 90 and telemetry circuitry 96 are described as separate circuitry, in some examples, processor circuitry 90 and telemetry circuitry 96 are functionally integrated. In some examples, processor circuitry 90 and telemetry circuitry 96 and telemetry circuitry 58 correspond to individual hardware units, such as microprocessors, ASICs, DSPs, FPGAs, or other hardware units. In other examples, any of processor circuitry 90 and telemetry circuitry 96 and telemetry circuitry 58 may correspond to multiple individual hardware units, such as microprocessors, ASICs, DSPs, FPGAs, or other hardware units.

Memory 92 may store program instructions that, when executed by processor circuitry 90, cause processor circuitry 90 and external programmer 24 to provide the functionality ascribed to external programmer 24 throughout this disclosure. In some examples, memory 92 may further include program information, e.g., stimulation programs defining the neurostimulation, similar to those stored in memory 56 of IMD 32. The stimulation programs stored in memory 92 may be downloaded into memory 56 of IMD 32.

In certain examples, the system includes a user interface 94 that allows patient 14 to provide input. IMD 32 may respond to patient-supplied data from the user interface by altering therapy. For example, patient 14 may use external programmer 24 (e.g., a handheld device) to record (by pushing a button) a physiological event of interest. Processor circuitry 53 of IMD 32 may respond by turning the therapy on or off and exiting or entering hibernation mode, or by adjusting the therapy (e.g., the stimulation strength) or by changing the therapy program. With reference to the urological applications discussed herein, patient 14 could push a button on external programmer 24 (e.g., their smartphone) when the bladder is voided. For example, this may send a signal to IMD 32 to turn off and enter hibernation mode for a period of time that is either pre-programmed based on the voiding characteristics of patient 14.

User interface 94 may include a button or keypad, lights, a speaker for voice commands, a display, such as a liquid crystal (LCD), light-emitting diode (LED), or cathode ray tube (CRT). In some examples the display may be a touch screen. As discussed in this disclosure, processor circuitry 90 may present and receive information relating to electrical stimulation and resulting therapeutic effects via user interface 94. For example, processor circuitry 90 may receive patient input via user interface 94. The input may be, for example, in the form of pressing a button on a keypad or selecting an icon from a touch screen.

Processor circuitry 90 may also present information to patient 14 in the form of alerts related to delivery of the electrical stimulation to patient 14 or a caregiver, as described in more detail below, via user interface 94. Although not shown, external programmer 24 may additionally or alternatively include a data or network interface to another computing device, to facilitate communication with the other device, and presentation of information relating to the electrical stimulation and therapeutic effects after termination of the electrical stimulation via the other device.

Telemetry circuitry 96 supports wireless communication between IMD 32 and external programmer 24 under the control of processor circuitry 90. Telemetry circuitry 96 may also be configured to communicate with another computing device via wireless communication techniques, or direct communication through a wired connection. In some examples, telemetry circuitry 96 may be substantially similar to telemetry circuitry 58 of IMD 32 described above, providing wireless communication via a radio frequency or proximal inductive medium. In some examples, telemetry circuitry 96 may include an antenna, which may take on a variety of forms, such as an internal or external antenna.

Examples of local wireless communication techniques that may be employed to facilitate communication between external programmer 24 and another computing device include RF communication according to the 802.11 or Bluetooth specification sets, infrared communication, e.g., according to the IrDA standard, or other standard or proprietary telemetry protocols. In this manner, other external devices may be capable of communicating with programmer 24 without needing to establish a secure wireless connection.

Power source 98 delivers operating power to the components of programmer 24. Power source 98 may include a battery and a power generation circuit to produce the operating power. In some examples, the battery may be rechargeable to allow extended operation.

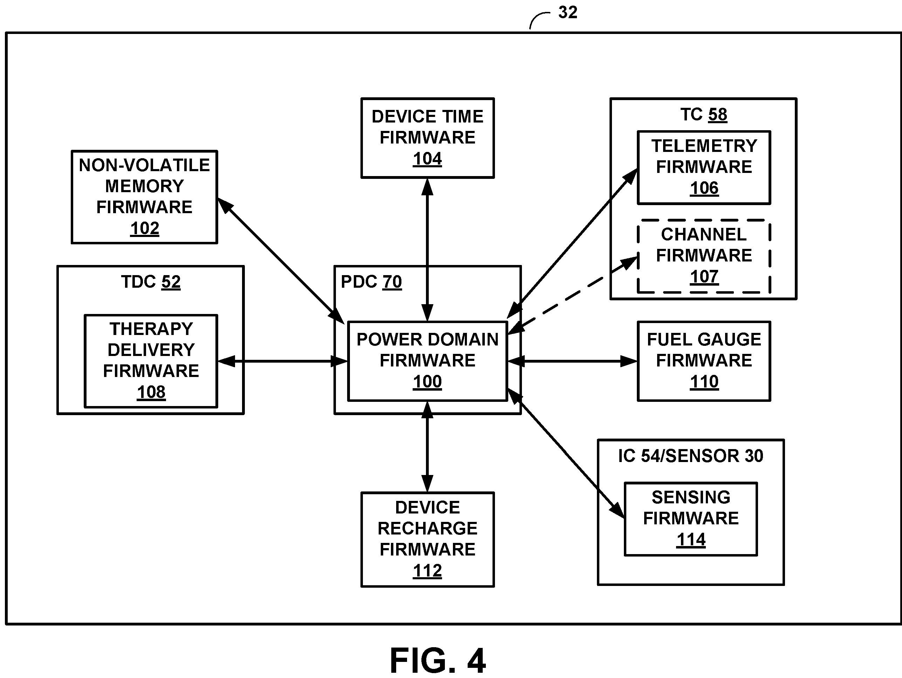

FIG. 4 is a block diagram illustrating an example neurostimulation device having a plurality of firmware modules according to the techniques of this disclosure. IMD 32 includes power domain firmware 100. Power domain firmware 100 may execute within power domain circuitry 70. IMD 32 may include a plurality of firmware modules. For example, IMD 32 may include non-volatile memory firmware 102, device time firmware 104, telemetry firmware 106, therapy delivery firmware 108, fuel gauge firmware 110 (which may be configured to provide an estimate of remaining charge of the battery powering IMD 32), and/or device recharge firmware 112 (which may be configured to manage battery recharge sessions), each of which may be referred to as a firmware module, whether they are implemented separately or together as one or more pieces of firmware. In some examples, telemetry circuitry of IMD 32 may be configured to use a plurality of channels. In such cases, IMD 32 may also include channel firmware 107 which may be associated with the plurality of channels. In examples, where IMD 32 includes sensing circuitry, such as impedance circuitry 54 and/or sensor 30 of FIG. 2, IMD 32 may include sensing firmware 114. While not depicted in FIG. 4, IMD 32 may include corresponding hardware (e.g., circuitry) for one or more of the plurality of firmware modules. For example, IMD 32 may include non-volatile memory 74 (FIG. 2) which may correspond to non-volatile memory firmware 102, telemetry circuitry 58 which may correspond to and execute telemetry firmware 106, therapy delivery circuitry 52 which may correspond to and execute therapy delivery firmware 108, etc. In some examples, there may not be corresponding hardware to particular firmware modules or multiple firmware modules may use a same group of hardware (e.g., circuitry). While specific firmware modules are depicted in FIG. 4, in some examples, fewer, more, or different firmware modules may be included in a device according to the techniques of this disclosure.

In some examples, IMD 32 may be a neurostimulator that includes power domain hardware capabilities that facilitate the ability to hibernate firmware features on IMD 32 to save power. IMD 32 may include firmware (e.g., power domain firmware 100) that allocates and manages power consumption requests to ensure IMD 32 has power available when needed, but hibernates as much as possible. For example, IMD 32 firmware logic may enable prioritized scheduling and timing of firmware features. Unlike other techniques of entering or exiting a hibernation mode, an implantable neurostimulator, such as IMD 32, may include a plurality of firmware modules (e.g., non-volatile memory firmware 102, device time firmware 104, telemetry firmware 106, therapy delivery firmware 108, fuel gauge firmware 110, device recharge firmware 112, and/or other firmware modules) executing on processing circuitry (e.g., processor circuitry 53, telemetry circuitry 58, therapy delivery circuitry 52, and a power domain firmware module 100 executing on power domain circuitry 70. Each of the plurality of firmware modules are configured to perform respective functions of the device (e.g., therapy delivery, telemetry, etc.). At least two of the plurality of firmware modules are configured to determine whether power is needed for a corresponding hardware component (e.g., therapy delivery circuitry 52, telemetry circuitry 58, etc.) or to perform the respective function during a respective time period. At least two of the plurality of firmware modules are also configured to generate and transmit one or more respective requests based on the determination of whether power is needed for the corresponding hardware component or to perform the respective function during the respective time period. Power domain firmware 100 is configured to receive the one or more respective requests and determine whether to open battery switch 72 or to keep battery switch 72 closed in response to the one or more respective requests. Power domain firmware 100 is also configured to control battery switch 72 to open or keep closed in response to the determination.

Power domain firmware 100 may be configured to receive input from a plurality of firmware modules (e.g., non-volatile memory firmware 102, device time firmware 104, telemetry firmware 106, therapy delivery firmware 108, fuel gauge firmware 110, device recharge firmware 112, and/or other firmware modules not shown in FIG. 4) regarding whether battery switch 72 may be opened or remain closed by power domain firmware 100. Power domain firmware 100 may moderate, mediate, or arbitrate, based on the input from the plurality of firmware modules, whether to open battery switch 72 (FIG. 2) or not and for how long to open battery switch 72.

For example, power domain firmware 100 may receive a request from telemetry firmware 106 to prevent battery switch 72 from being opened until further notice and may receive a request from therapy delivery firmware 108 that allows battery switch 72 to be opened until further notice. In such a case, power domain firmware 100 may determine to keep battery switch 72 closed and control the battery switch to remain closed because telemetry firmware 106 has indicated that battery switch 72 should remain closed. In such an example, telemetry firmware 106 and/or telemetry circuitry 58 still require power, for example, to perform telemetry operations. In another example, power domain firmware 100 may receive a request from therapy delivery firmware 108 to prevent battery switch 72 from being opened until a first time period expires (e.g., 5 minutes from now) and a request from telemetry firmware 106 to allow battery switch 72 to be opened now, but require battery switch 72 to be closed when a second time period expires (e.g., 5 minutes from now). In such an example, power domain firmware 100 may determine to keep battery switch 72 closed and control the battery switch to remain closed until at least power domain firmware 100 receives a request from telemetry firmware 106 that permits the opening of battery switch 72. In such a manner, power domain firmware 100 does not control battery switch 72 to open unless all received requests indicate that it is okay to open battery switch 72 and then only so long as all received requests indicate that it is okay to keep battery switch 72 opened.

Power domain firmware 100 is configured to control battery switch 72 to open or to close based on the requests, such as firmware requests or hardware interrupts, from the plurality of firmware modules. In this manner, power domain firmware 100 may act as an arbiter or mediator of the requests received from the plurality of firmware modules. For example, when battery switch 72 is closed, the battery (e.g., power source 60) of IMD 32 may provide power to the components of IMD 32 requiring power and IMD 32 may be said to be in an operational mode. When battery switch 72 is open, the battery provides power to less than all components of IMD 32 that require power to operate, thereby reducing power consumption. In such a case, IMD 32 may be said to be in a hibernation mode.

In some examples, power domain firmware 100 may store information about why battery switch 72 was closed and why battery switch 72 was opened. In some examples, power domain firmware 100 may send a message to the plurality of firmware modules indicating why battery switch 72 is about to be opened prior to opening battery switch 72. In some examples, rather than opening battery switch 72, power domain firmware 100 may send a message instructing power domain circuitry 70 (FIG. 2) to open battery switch 72 and power domain circuitry 70 may open battery switch 72 in response to the message.

In some examples, power domain firmware 100 may perform an analysis of the received requests from the plurality of firmware modules on a periodic basis. For example, power domain firmware 100 may perform such an analysis every predetermined amount of time. Such a predetermined amount of time may include 5 seconds, 10 seconds, 15 seconds, 16 seconds, 20 second, 30 seconds, or any other predetermined amount of time. In other examples, power domain firmware 100 may perform the analysis of the received requests continuously, in real-time. The results of such an analysis may be to open battery switch 72 and, in some examples, for how long to keep battery switch 72 open. Alternatively, the results of such an analysis may be to keep battery switch 72 closed. In some examples, the maximum length of time battery switch 72 may be open may be limited. By limiting the maximum length of time battery switch 72 may be open, IMD 32 may be in an operational mode when incoming telemetry or therapy delivery is expected.

In some examples, power domain firmware 100 may send a notification to each of the plurality of firmware modules prior to opening battery switch 72 to allow time for data used by the plurality of firmware modules to be stored in non-volatile memory 74 (FIG. 2) prior to opening battery switch 72, as opening battery switch 72 may cause the loss of data if the data is only stored in volatile memory, such as RAM. In some examples, power domain firmware 100 may commit the data from the plurality of firmware modules to non-volatile memory 74, rather than each of the plurality of firmware modules performing the commitment. In some examples, when battery switch 72 is re-closed, each of the plurality of firmware modules may retrieve their own stored data from non-volatile memory 74. The notification that the switch is going to be opened may also allow any of the plurality of firmware modules to send a request to keep battery switch 72 closed prior to battery switch 72 being opened.

In some examples, power domain firmware 100 allows other parts of firmware (e.g., the plurality of firmware modules) to have input into when a battery switch may be opened and closed. In some examples, power domain firmware 100 may receive from any of the plurality of firmware modules a scheduling request. This scheduling request may be limited to one of four types of requests. For example, at least two of the plurality of firmware modules (not including power domain firmware 100) may transmit a request to power domain firmware 100 during times when the plurality of firmware modules are powered. Such a request may include: a) allow battery switch 72 to open at any time until further notice; b) do not allow (e.g., prevent) battery switch 72 to open at all until further notice; c) do not allow (e.g., prevent) battery switch 72 to open until X seconds from now; or d) allow battery switch 72 to open now, but the close the switch and have firmware fully operational in Y seconds.

Power domain firmware 100 may periodically (or, alternatively, continuously) evaluate all the requested conditions from the plurality of firmware modules, and if all of the received requests by power domain firmware 100 indicate that battery switch 72 can open immediately, power domain firmware 100 may open battery switch 72 for an appropriate length of time. This appropriate length of time may be based on the received requests from the plurality of firmware modules. For example, if therapy delivery firmware 108 requests that battery switch 72 can be open, but needs to be closed and have firmware fully operational in 8 seconds and any other received requests indicate that battery switch 72 may be opened for those 8 seconds, power domain firmware 100 may open battery switch 72 and power domain circuitry 70 may close battery switch 72 at a time such that therapy delivery firmware 108 is fully operational 8 seconds after receiving the request from therapy delivery firmware 108. It should be noted that this 8 second period may be shortened by the detection of incoming telemetry or battery recharge energy. For example, if, after opening battery switch 72, IMD 32 detects incoming telemetry or battery recharge energy, power domain circuitry 70, telemetry circuitry 58, or other circuitry may close the switch in response to detecting the incoming telemetry or recharge energy before the 8 seconds has expired.

In some examples, power domain firmware 100 may maintain lifetime device values which any of the plurality of firmware modules may reference as needed or as is desirable. For example, power domain firmware 100 may maintain a lifetime count of a cumulative amount of time that IMD 32 had battery switch 72 open and a lifetime count of a cumulative amount of time that IMD 32 had battery switch 72 closed. A lifetime count may include a count over a lifetime of the device (e.g., IMD 32).

Non-volatile memory firmware 102 may manage the power needs of non-volatile memory 74 (FIG. 2) by generating and sending requests to power domain firmware 100. Non-volatile memory 74 may be configured to store data in a manner such that when unpowered, the data is preserved even when non-volatile memory 74 or other components of IMD 32 are not powered. In some examples, this data may include diagnostic information, such as mirrors and logs, which may be stored in state and memory 56. Non-volatile memory firmware 102 may be configured to track a standard device time. In some examples, this standard device time may include a total number of device resets and a count of seconds after the most recent reset. In some examples, non-volatile memory firmware 102 may also maintain a diagnostic log of a predetermined number (e.g., 10) of most recent resets.

Fuel gauge firmware 110 may be configured to use voltage measurements, coulomb counter ticks, and a battery charge curve to provide an estimate of remaining battery charge. Device recharge firmware 112 may be configured to manage battery recharge sessions.

Power domain circuitry 70 may close an open battery switch 72 due to incoming telemetry, incoming recharge energy, or after a defined interval (e.g., the time period determined by power domain firmware 100 to keep the battery switch open). Power domain firmware 100 may be configured to reliably and robustly determine the duration battery switch 72 was open, regardless of how battery switch 72 was closed. If requests from one or more of the plurality of firmware modules indicate that battery switch 72 should be prevented from opening, any preparatory actions taken by power domain firmware 100 to open battery switch 72 may be undone so that the one or more of the plurality of firmware module issuing the request(s) will continue to be powered and can continue operation.

For example, non-volatile memory 74 may request that battery switch 72 remain closed while non-volatile memory 74 is updating the diagnostic information. At other times, non-volatile memory 74 may not issue a request to keep the battery switch closed. For example, non-volatile memory 74 may not require power during times that diagnostic information is not being updated. As such, non-volatile memory 74 may issue a request to open the switch. Prior to opening battery switch 72, all important data may be written to non-volatile memory 74 for persistent storage during a power down event. In some examples, because it may be desirable that no new data is generated after the important data is written to non-volatile memory 74 before battery switch 72 opens, IMD 32 may prevent any firmware module to execute normally after the data is written to non-volatile memory 74 and before battery switch 72 is opened.

Device time firmware 104 may update a device time, which power domain firmware 100 may store in a mirror in non-volatile memory 74 just before the battery switch opens. If battery switch 72 does not open for some reason, a device time system (not shown), of which device time firmware 104 may be a part, may be restarted.

Telemetry firmware 106 may require battery switch 72 to be closed during downlink, processing, or uplink of any telemetry message as telemetry circuitry 58 may otherwise be powered off. In some examples, telemetry firmware 106 may require battery switch 72 to be closed for a period of time after each telemetry message and may issue an appropriate request to keep the battery switch closed until a first time period expires. In other words, telemetry firmware 106 may issue a request to prevent battery switch 72 from being opened until the first time period expires. In other cases, telemetry firmware 106 may issue a request to allow battery switch 72 to be opened at any time until further notice. In some examples, telemetry firmware 106 may make use of power domain hardware (e.g., of power domain circuitry 70 of FIG. 2) to detect incoming telemetry, while maintaining control of the telemetry. In some examples, power domain firmware 100 may be powered down when battery switch 72 is open. In such examples, power domain circuitry 70 may be configured to close battery switch 72 when incoming telemetry is detected. For example, power domain circuity 70 may detect energy on an antenna or coil used for telemetry to detect incoming telemetry.

Therapy delivery firmware 108 may request battery switch 72 to remain closed any time that stimulation is being delivered. Therapy delivery firmware 108 may issue a request allowing battery switch 72 to open any time that therapy is off or during long cycle-off durations when therapy is on, but no stimulation is currently being delivered. If battery switch 72 is to be opened during long cycle-off periods, therapy delivery firmware 108 may issue a request to allow the battery switch to be opened now, but to require the battery switch to be closed when a second time period expires. In this manner, battery switch 72 may be closed and therapy delivery firmware 108 (and/or other firmware modules) may be fully restarted before the next cycle-on period. Similarly, if battery switch 72 is opened during long cycle-off periods, battery switch 72 may be closed and therapy delivery firmware 108 (and/or other firmware modules) may be fully restarted before the next cycle-on period. In some examples, therapy delivery firmware 108 may perform some clean-up actions before battery switch 72 is opened. When battery switch 72 closes and therapy delivery firmware 108 restarts, therapy delivery firmware 108 may use saved therapy data and the lifetime counts of cumulative amount of open/closed switch times to determine when therapy delivery should resume.

Fuel gauge firmware 110 may issue a request that allows power domain firmware 100 to open battery switch 72 at any time. When fuel gauge firmware 110 is running, battery discharge may be tracked in at least two ways: via a coulomb counter interrupt that occurs every predetermined amount of battery discharge; or via a firmware timer used to account for overhead discharge not measured by hardware. When power domain firmware 100 is about to open battery switch 72, fuel gauge firmware 110 may read a coulomb counter register (not shown) to see how much charge has been used since the last coulomb counter interrupt. The read information may be saved to a power mirror in non-volatile memory 74 (FIG. 2) to be added to the discharge total later. Fuel gauge firmware 110 may also read an overhead discharge firmware timer and save the read information to the power mirror so the information can be used when restarting the timer when fuel gauge firmware 110 restarts. In some examples, when battery switch 72 closes, fuel gauge firmware 110 may use the switch open duration to estimate the discharge that occurred while battery switch 72 was open. For example, a technician may determine, for example, in a laboratory, the amount of discharge that occurs over time with IMD 32 and fuel gauge firmware 110 may use such information to estimate the discharge that occurred while battery switch 72 was open.

Device recharge firmware 112 may need battery switch 72 to remain closed during a commanded recharge session or during an automatic passive recharge session which occurs when a non-timed non-power domain firmware 100 battery switch closure occurs. In some examples, when device recharge firmware 112 issues a request to keep battery switch 72 closed, IMD 32 may determine that the battery voltage is very low and IMD 32 needs to be recharged. In some examples, IMD 32 is configured to accept recharge energy and remain in a powered, battery switch 72 closed mode for several minutes or until a first telemetry command is received. As it may be inappropriate for battery switch 72 to be opened during a battery recharge session or during an active telemetry session. Therefore, device recharge firmware 112 and/or telemetry firmware 106 may be configured to request that the battery switch remain closed (e.g., thereby preventing the battery switch from opening) until after the recharge session ends and/or after the telemetry session is complete.

For example, power domain firmware 100 may not allow battery switch 72 to open, for example, until 10 seconds from now when power domain firmware 100 first starts so as to give the firmware enough time to initialize and telemetry to be received before battery switch 72 is opened again. For example, telemetry firmware 106 may issue a request that power domain firmware 100 not allow battery switch 72 to be opened until, for example, 60 seconds from now. Telemetry firmware 106 may do this every time a telemetry message is received. By issuing such a request, telemetry firmware 106 may ensure battery switch 72 is closed and telemetry firmware remains ready for more telemetry messages. For example, therapy delivery firmware 108 may issue a request that power domain firmware 100 allow battery switch 72 to be opened at any time until further notice when therapy delivery firmware 108 is not delivering therapy. Therapy delivery firmware 108 may issue a request to prevent battery switch 72 from being opened until further notice, for example, when therapy is being delivered. Therapy delivery firmware 108 may issue a request to allow battery switch 72 to be opened now, but to require battery switch 72 to be closed when a second time period expires, for example, when therapy is enabled at the beginning of a long cycle-off period or before a therapy delivery session that will occur at a later point in time.

For example, device recharge firmware 112 may issue a request to prevent battery switch 72 from reopening until further notice. Device recharge firmware 112 may issue such a request at the beginning of a commanded recharge session. Device recharge firmware 112 may issue a request to allow battery switch 72 to be opened at any time until further notice at the end of a commanded recharge session or at the end of an automatic passive recharge session. Device recharge firmware 112 may issue a request to prevent battery switch 72 from opening until, for example, 120 seconds from now, when starting an automatic passive recharge session which occurs when a non-timed non-power domain firmware 100 battery switch closure occurs.

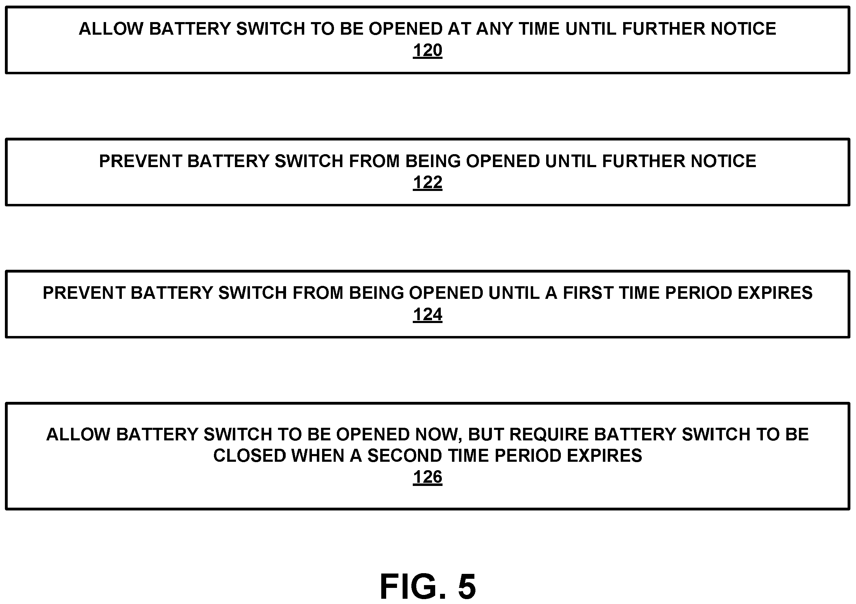

FIG. 5 is a conceptual diagram illustrating example requests which a plurality of firmware modules may send to power domain firmware. For example, at least two of the plurality of firmware modules may send one or more requests to power domain firmware 100.

The requests of FIG. 5 may include a first request 120 to allow battery switch 72 to be opened at any time until further notice. For example, therapy delivery firmware 108 may send first request 120 to power domain firmware 100 to when therapy delivery functionality of IMD 32 is off. The requests may also include a second request 122 to prevent battery switch 72 from being opened until further notice. For example, therapy delivery firmware 108 may send second request 122 to power domain firmware 100 when therapy is being actively delivered by IMD 32.

The requests may also include a third request 124 to prevent battery switch 72 from being opened until a first requested time period expires. In some examples, this first time period may be a specified, requested amount of time (e.g., by a firmware module). In other examples, the time period may be a predetermined amount of time. For example, telemetry firmware 106 may send third request to power domain firmware 100 after receiving an incoming telemetry message to keep telemetry circuitry 58 and processor circuitry 53 powered up in case another incoming telemetry message is received or in case telemetry circuitry 58 has to respond to the incoming telemetry message. As mentioned above, in some examples, the time period is predetermined and not necessarily contained within third request 124. For example, the predetermined time period may be 60 seconds. In some examples, the time period is specified by the firmware sending the request. For example, telemetry firmware 106 may send third request 124 including a time period during which telemetry firmware 106 indicates that battery switch 72 should be closed or remain closed, for example, 60 seconds.

The requests may also include a fourth request 126 to allow battery switch 72 to be opened now, but require that battery switch 72 be closed when a second requested time period expires. In some examples, this second requested time period may be a specified, requested amount of time (e.g., by a firmware module). In some examples, the time period may be a predetermined amount of time. For example, therapy delivery firmware 108 may send fourth request 126 to power domain firmware 100 when therapy delivery is scheduled to begin at the end of the second specified time or the second predetermined time. As mentioned above, in some examples, the time period is predetermined and not necessarily contained within fourth request 126. For example, the predetermined time period may be 10 minutes. In some examples, the time period is specified by the firmware sending the request. For example, therapy delivery firmware 108 may send fourth request 126 including a time period during which therapy delivery firmware 108 indicates that battery switch 72 should be closed or remain closed, for example, 10 minutes seconds. The first time period may be the same as, or different than, the second time period.

In some examples, the plurality of firmware modules may only send first request 120, second request 122, third request 124, and/or fourth request 126 to power domain firmware 100. In other words, in some examples, each request sent to power domain firmware 100 by any of the plurality of firmware modules is selected from a list consisting of first request 120, second request 122, third request 124, and fourth request 126. For example, power domain firmware 100 may receive from the plurality of firmware modules one or more requests. In some examples, these one or more requests may be selected, by one or more of the plurality of firmware modules, from a list consisting of: a) a request to allow battery switch 72 to be opened at any time until further notice, b) a request to prevent battery switch 72 from being opened (or keep battery switch 72 closed) until further notice, c) a request to prevent battery switch 72 from being opened (or keep battery switch 72 closed) until a first time period expires, or d) a request to allow battery switch 72 to be opened now, but to require battery switch 72 to be closed when a second time period expires.

For example, power domain firmware 100 may receive second request 122 from telemetry firmware 106 to prevent battery switch 72 from being opened until further notice therapy delivery circuitry that allows battery switch 72 to be opened until further notice. In such a case, power domain firmware 100 may determine to keep battery switch 72 closed and control the battery switch to remain closed because telemetry firmware 106 has indicated that battery switch 72 should remain closed. In such an example, telemetry firmware 106 and/or telemetry circuitry 58 still require power, for example, to perform telemetry operations. In another example, power domain firmware 100 may receive a request 124 from therapy delivery firmware 108 to prevent battery switch 72 from being opened until a first time period expires (e.g., 5 minutes from now) and a request 126 from telemetry firmware 106 to allow battery switch 72 to be opened now, but require battery switch 72 to be closed when a second time period expires (e.g., 5 minutes from now). In such an example, power domain firmware 100 may determine to keep battery switch 72 closed and control the battery switch to remain closed until at least power domain firmware 100 receives a request from telemetry firmware 106 that permits the opening of battery switch 72. In such a manner, power domain firmware 100 does not control battery switch 72 to open unless all received requests indicate that it is okay to open battery switch 72 and then only so long as all received requests indicate that it is okay to keep battery switch 72 opened.

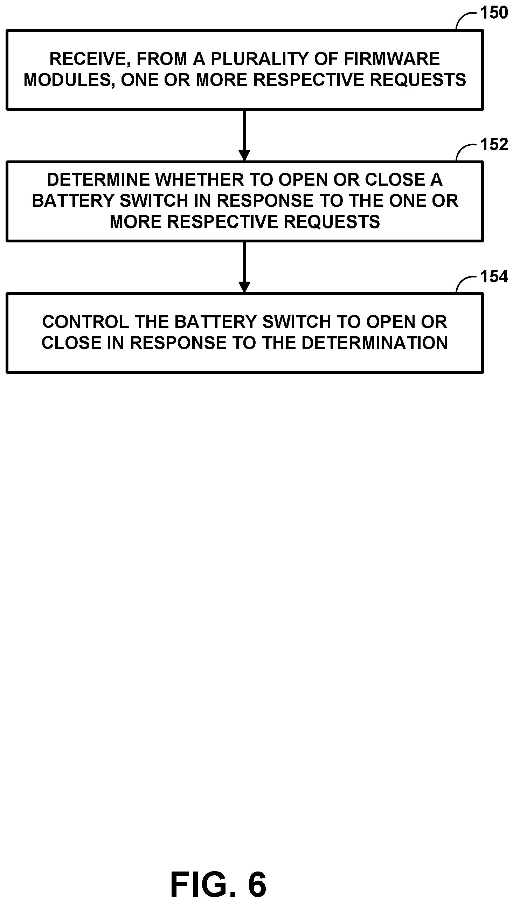

FIG. 6 is a flow diagram illustrating example battery switch control techniques according to one or more aspects of this disclosure. While described primarily with respect to IMD 32 of FIG. 4, the techniques of FIG. 6 may be performed by any battery powered device having a battery switch.

Power domain firmware 100 may receive, from a plurality of firmware modules, one or more respective requests (150). For example, power domain firmware 100 may receive requests from any of non-volatile memory firmware 102, device time firmware 104, telemetry firmware 106, therapy delivery firmware 108, fuel gauge firmware 110, device recharge firmware 112, and/or other firmware modules not depicted in FIG. 4. In some examples, the one or more respective requests include at least one of a) a request to allow battery switch 72 to be opened at any time until further notice, b) a request to prevent battery switch 72 from being opened until further notice, c) a request to prevent battery switch 72 from being opened until a first time period expires, or d) a request to allow battery switch 72 to be opened now, but to require battery switch 72 to be closed when a second time period expires.

Power domain firmware 100 may determine whether to open battery switch 72 in response to the one or more respective requests (152). For example, power domain firmware 100 may periodically or continuously monitor for requests and evaluate the requests to determine whether each request which power domain firmware 100 has received permit the opening of battery switch 72 or if any of the received requests require that battery switch 72 remain closed.

Power domain firmware 100 may control battery switch 72 to open in response to the determination (154). For example, if all of the received requests permit the opening of battery switch 72 at a first given time, power domain firmware 100 (or power domain circuitry 70) may control battery switch 72 to open. If at least one of the received requests require that battery switch 72 be kept closed, power domain firmware 100 may control battery switch 72 to remain closed. In some examples, power domain firmware 100 controls battery switch 72 via power domain circuitry 70 rather than directly control battery switch 72. For example, when battery switch 72 is to be opened, power domain firmware 100 may directly open battery switch 72 or may send a message to power domain circuitry 70 to open battery switch 72 and, in some examples, for how long to open battery switch 72.

In some examples, the one or more respective requests each permit battery switch 72 to be opened and, in those examples, the determination is to open the battery switch. In some examples, at least one of the one or more respective requests comprises a request to prevent the battery switch from opening and wherein the determination is to keep the battery switch closed.

In some examples, power domain firmware 100 is further configured to control the battery switch 72 to open only during a given time period when each of the plurality of firmware modules have not requested that battery switch 72 be prevented from being opened during the given time period. In some examples, the plurality of firmware modules includes at least one of the following firmware modules: non-volatile memory firmware; device timer firmware; fuel gauge firmware; device recharger firmware; a therapy delivery firmware; or telemetry firmware.

In some examples, therapy delivery circuitry 52 (FIG. 2) generates an electrical stimulation signal and telemetry circuitry 58 communicates with another device (e.g., external programmer 24 of FIG. 1C). In some examples, the power domain firmware further configures the processing circuitry to determine a maximum duration the battery switch remains open in response to the one or more respective requests. In some examples, power domain firmware 100 stores in memory a lifetime count of a cumulative amount of time battery switch 72 is closed and a lifetime count of a cumulative amount of time battery switch 72 is open.

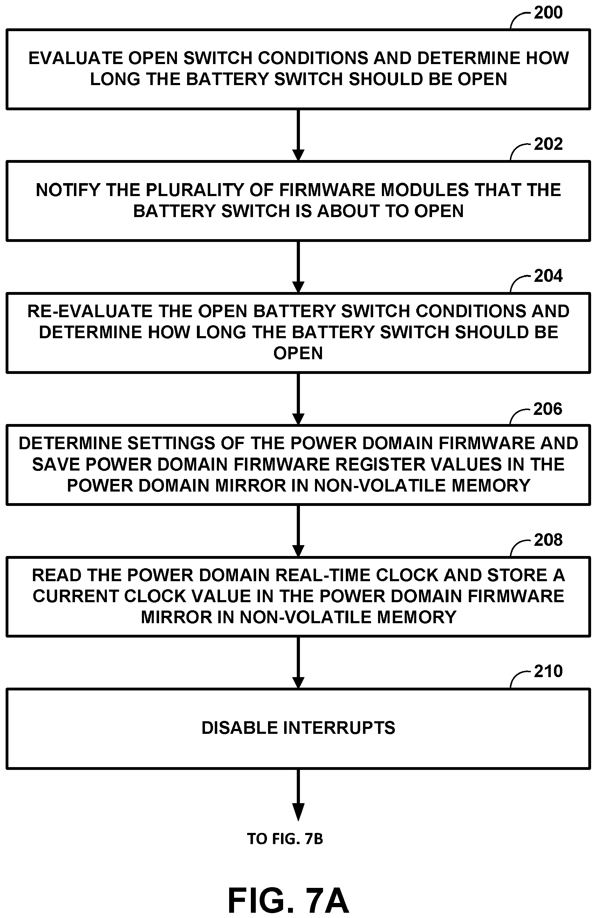

FIGS. 7A-7D are flow diagrams illustrating an example technique for opening a battery switch. While described primarily with respect to IMD 32 of FIG. 4, the techniques of FIGS. 7A-7D may be performed by any battery powered device having a battery switch.

Power domain firmware 100 may evaluate open switch conditions and determine how long battery switch 72 should be open (200). For example, power domain firmware 100 may determine that all received requests from the plurality of firmware modules permit battery switch 72 to be open. If one or more received requests from the plurality of firmware modules do not permit battery switch 72 to be open, power domain firmware 100 may not open battery switch 72 at this time.

Power domain firmware 100 may notify the plurality of firmware modules that battery switch 72 is about to open (202). For example, power domain firmware 100 may send a message to each of the plurality of firmware modules to notify the plurality of firmware modules that battery switch 72 is about to open. By notifying the plurality of firmware modules about the impending opening of battery switch 72, power domain firmware 100 may provide each of the plurality of firmware modules an opportunity to evaluate the need for power and possibly send a new request to power domain firmware 100 to prevent battery switch 72 from opening. This notification also provides each of the plurality of firmware modules to read module-specific partially updated data and prepare for shutdown. Each of the plurality of firmware modules may update logs and mirrors as needed to ensure that all data can be recovered and operations continued once battery switch 72 is closed and power is restored.

Power domain firmware 100 may re-evaluate the open battery switch conditions and determine how long battery switch 72 should be open (204). For example, in response to notifying the plurality of firmware modules about the impending opening of battery switch 72, power domain firmware 100 may receive further request(s) from any of the plurality of firmware modules which may warrant a re-evaluation or change the length of time battery switch 72 should be open. If the re-evaluation indicates that battery switch 72 should not be opened, power domain firmware 100 may send a notification to each of the plurality of firmware modules that battery switch 72 will not be opened at this time and cancel the battery switch opening operation.

Power domain firmware 100 may determine settings of the power domain firmware 100 (including, for example, timer settings) and save the power domain firmware 100 register values that will be used in the power domain firmware 100 mirror in non-volatile memory 74 (206), as these settings and values may be used later when battery switch 72 closes again.

Power domain firmware 100 may read a power domain firmware 100 real-time clock and store the current value of the power domain firmware 100 real-time clock in the power domain firmware 100 mirror in non-volatile memory 74 (208). The real-time clock value may be used later when battery switch 72 closes again. In some examples, if an error occurred reading the real-time clock, power domain firmware 100 may store a zero value for the real-time clock in the power domain firmware 100 mirror in non-volatile memory 74.

Power domain firmware 100 may disable interrupts (210). For example, power domain firmware 100 may block other module (e.g., hardware and/or firmware) from interrupting the open battery switch process.

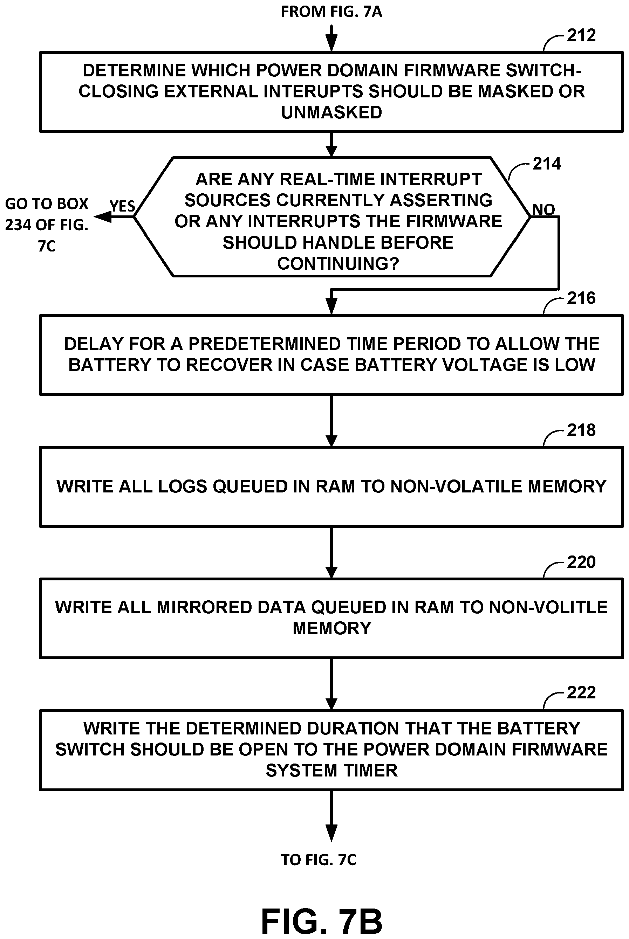

As shown in FIG. 7B, power domain firmware 100 may determine which power domain firmware 100 switch-closing external interrupts should be masked or unmasked (212). For example, power domain firmware 100 may ignore a masked interrupt while battery switch 72 is open, but not ignore unmasked interrupts.

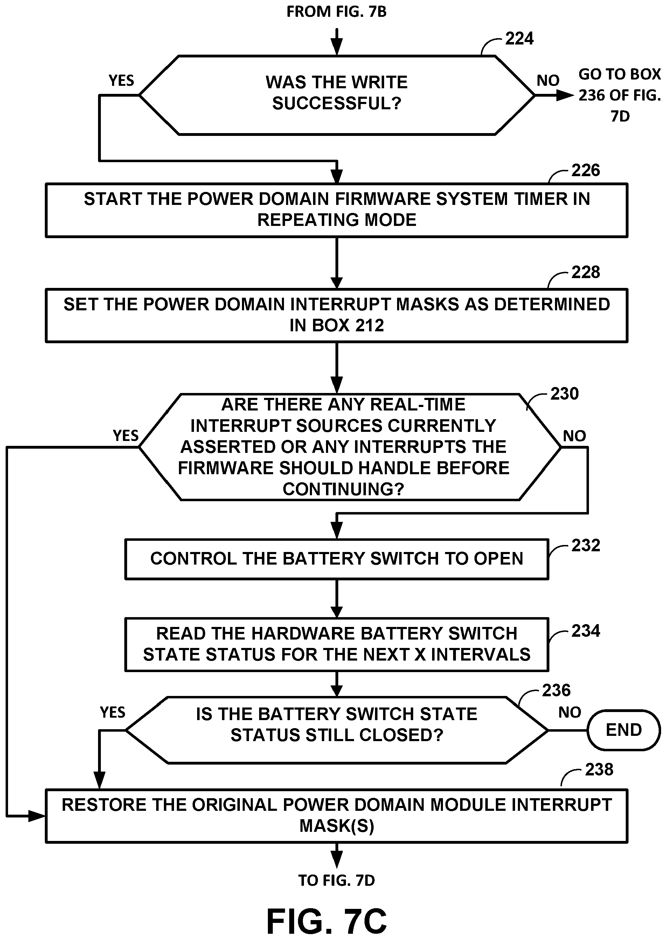

Power domain firmware 100 may determine whether there are any battery switch closure interrupt sources (e.g., real-time interrupt sources) currently asserting (214). If power domain firmware 100 determines that there are any real-time interrupt sources currently asserting or any interrupts power domain firmware 100 should handle before continuing (the "YES" path from box 214), power domain firmware 100 may proceed to box 234 of FIG. 7C and cancel the opening of battery switch 72. For example, power domain firmware may prevent the opening of battery switch 72 or may reassert a close battery switch event. This may be performed to avoid a situation where power domain firmware 100 opens battery switch 72 only to have power domain circuitry 70 immediately close battery switch 72.

If power domain firmware 100 determines that there are no real-time interrupt sources currently asserting or interrupts power domain firmware 100 should handle before continuing (the "NO" path from box 214), in some examples, power domain firmware 100 may delay opening battery switch 72 for a predetermined period of time, such as 5 ms, 10 ms, 20 ms, 30 ms, 40 ms, 50 ms, 60 ms, 70 ms, or other predetermined period of time, to allow the battery to recover in case the battery voltage is low. Power domain firmware 100, or any of the plurality of firmware modules, may write all logs queued in RAM to non-volatile memory 74 (218).

Power domain firmware 100, or any of the plurality of firmware modules, may write all data related to diagnostics or use that is queued in RAM to non-volatile memory 74 (220). In this manner, such data may be available when battery switch 72 is closed again.

Power domain firmware 100 may write the determined duration that battery switch 72 should be open (see boxes 200 and 204 of FIG. 7A) to the power domain firmware 100 system timer (222).

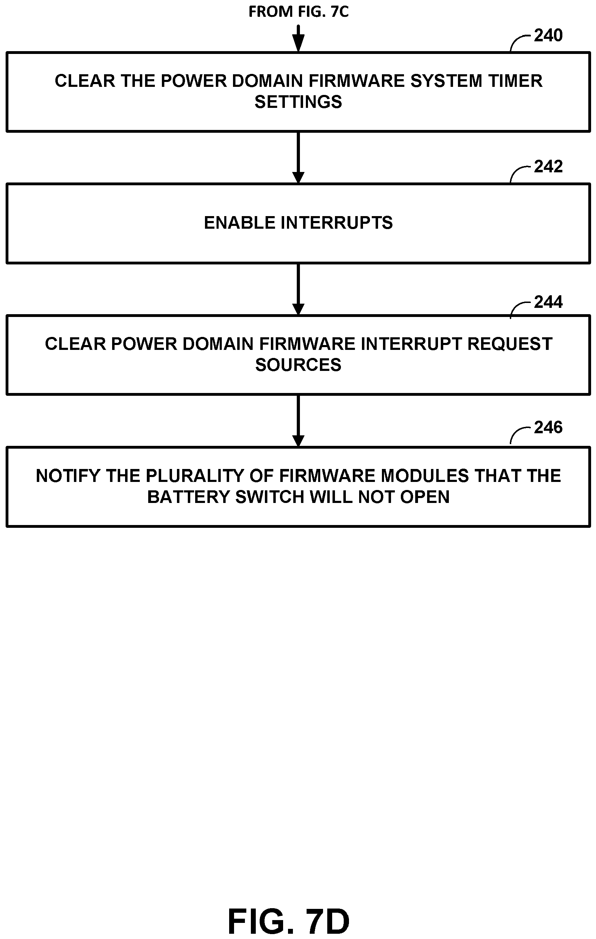

As shown in FIG. 7C, in some examples, power domain firmware 100 may determine whether the write operation was successful (224). If the write operation was not successful (the "NO" path from box 224), power domain firmware 100 may cancel the battery switch opening and cancel the whole process and go to box 236 of FIG. 7D.

If the write operation was successful (the "YES" path from box 224), power domain firmware 100 may start the power domain firmware 100 system timer in repeating mode (226). For example, repeating mode may be used for robustness. In repeating mode, if battery switch 72 is not closed when the power domain firmware 100 system timer expires, the power domain firmware 100 system timer will expire again until battery switch 72 closes.

Power domain firmware 100 may set the interrupt masks as determine in box 212 of FIG. 7B (228). Power domain firmware 100 may recheck to determine whether there are any battery switch closure interrupt sources currently asserting (230). If there are no real-time interrupt sources currently asserting or interrupts power domain firmware 100 should handle before continuing (the "NO" path from box 230), power domain firmware 100 may request battery switch 72 REo open (232). Power domain firmware 100 may delay for X seconds (234). Power domain firmware 100 may delay for a predetermined period of time, such as 5 ms, 10 ms, 20 ms, 30 ms, 40 ms, 50 ms, 60 ms, 70 ms, or other predetermined period of time. Power domain firmware 100 may determine whether the battery switch stat status is still closed (236). If the batter switch state status is not still closed (the "NO" path from box 236), the battery is open and the sequence may end. If the battery switch state status is still closed (the "YES" path from box 236), power domain firmware 100 may restore the original power domain firmware 100 interrupt mask(s) (238). For example, power domain firmware 100 may restore the power domain firmware 100 interrupt mask(s) that existed prior to setting the power domain interrupt mask(s) in box 228. In some examples, if battery switch 72 does not open due to the hardware and firmware of IMD getting out of sync, power domain firmware 100 may stay in a loop until a watchdog timer triggers a device reset.

If there are any real-time interrupt sources currently asserting or any interrupts power domain firmware 100 should handle before continuing (the "YES" path from box 230), power domain firmware 100 may restore the original power domain firmware 100 interrupt mask(s) (238).

As shown in FIG. 7D, power domain firmware 100 may clear the power domain firmware 100 system timer settings (240). Power domain firmware 100 may enable interrupts (242). For example, power domain firmware 100 may enable the currently unmasked interrupts. Power domain firmware 100 may clear power domain firmware interrupt request sources (244). For example, certain power domain firmware interrupt request sources may be byproducts of a battery switch close event and may be cleared. Power domain firmware 100 may notify the plurality of firmware modules that battery switch 72 will not open (246). In some examples, in response to receiving a notification that battery switch 72 will not open, the plurality of firmware modules each may undo any actions which they undertook to prepare for shutdown, such as restart the device time timer.

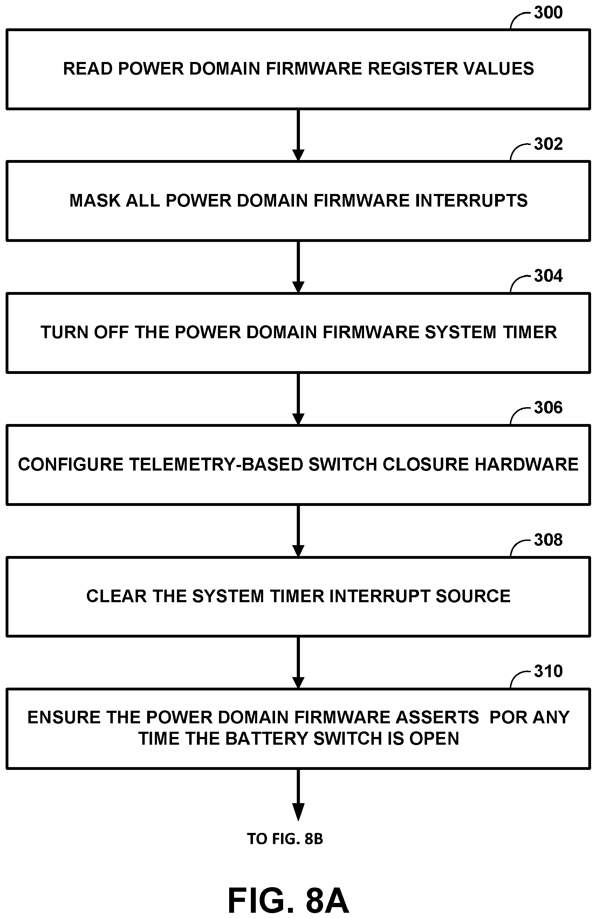

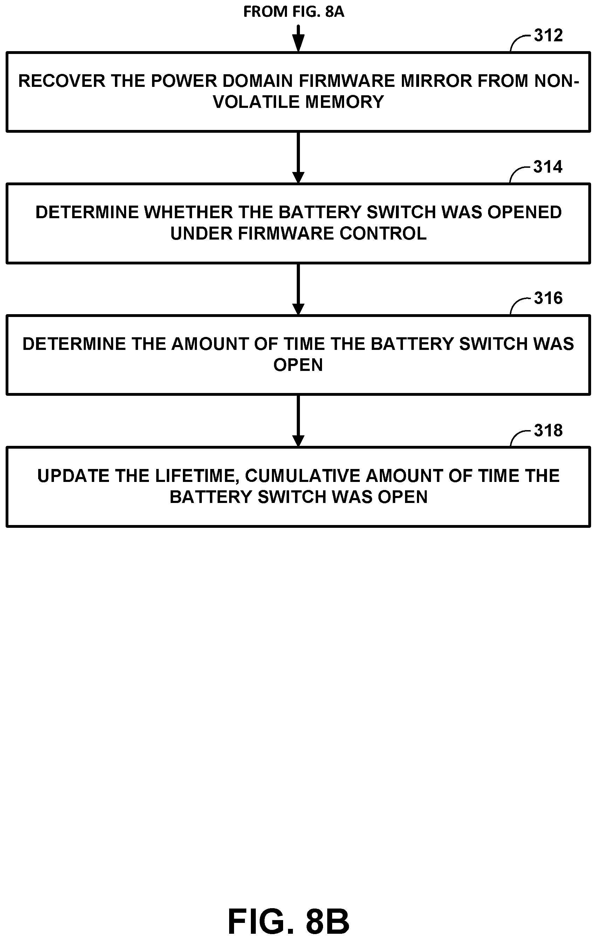

FIGS. 8A-8B are flow diagrams of an example technique for closing a battery switch according to one or more aspects of this disclosure. Power domain firmware 100 may read power domain firmware 100 register values (300), for example, from non-volatile memory 74. This may occur as soon as possible when the power domain firmware 100 firmware starts. Such value may be used in conjunction with the values (stored in the power domain firmware 100 mirror) that were written to the power domain firmware registers of non-volatile memory 74 before battery switch 72 was opened.

Power domain firmware 100 may mask all power domain firmware 100 interrupts (302). Such power domain firmware 100 interrupts may be unmasked as needed as firmware of IMD 32 continues to execute.

Power domain firmware 100 may turn off the power domain firmware 100 system timer (304). Power domain circuitry 70 may configure telemetry-based switch closure hardware (306). Power domain firmware 100 may clear the system timer interrupt source (308).

As shown in FIG. 7B, power domain firmware 100 may recover the power domain firmware 100 mirror from non-volatile memory 74 (312). For example, power domain firmware 100 may load the power domain firmware 100 mirror from non-volatile memory 74.

Power domain firmware 100 may determine whether battery switch 72 was opened under firmware control (314). For example, power domain firmware 100 may use the startup power domain firmware 100 register values and the saved power domain firmware 100 values from before battery switch 72 was opened to determine whether battery switch 72 was opened under firmware control.

Power domain firmware 100 may determine the number of seconds battery switch 72 was open (316). For example, power domain firmware 100 may use the power domain firmware 100 real-time clock to determine the number of seconds battery switch 72 was open. In some examples, if there was an error reading the power domain firmware 100 real time clock either before or after the opening battery switch 72, power domain firmware 100 may determine the number of seconds battery switch 72 was open from the startup value in the power domain firmware 100 system timer and the initial timer value set before battery switch 72 was opened.

Power domain firmware 100 may update the lifetime count of a cumulative amount of time battery switch 72 was open (318).

The plurality of firmware modules may continue with their recovery from the open battery switch situation. For example, therapy delivery firmware 108 may determine if and when therapy should be restarted and may schedule such a restart of therapy. Fuel gauge 110 may adjust the estimate of the state of charge based on the open-switch duration and/or the partial discharge coulomb counter information stored before battery switch 72 was opened. Firmware of non-volatile memory 74 may updated the reset diagnostic (or not) depending on whether battery switch 72 was opened for by power domain firmware 100 for power savings or was opened for another purpose, such as by being determined by the system design.

It should be noted that the techniques of this disclosure are not limited to IMDs or medical devices. These techniques may be applicable to any device having a battery and a battery switch. It should also be noted that system 10, and the techniques described herein, may not be limited to treatment or monitoring of a human patient. In alternative examples, system 10 may be implemented in non-human patients, e.g., primates, canines, equines, pigs, and felines. These other animals may undergo clinical or research therapies that my benefit from the subject matter of this disclosure.

The techniques of this disclosure may be implemented in a wide variety of computing devices, medical devices, or any combination thereof. Any of the described units, circuitry or components may be implemented together or separately as discrete but interoperable logic devices. Depiction of different features as circuitry or units is intended to highlight different functional aspects and does not necessarily imply that such circuitry or units must be realized by separate hardware, firmware, or software components. Rather, functionality associated with one or more circuitry or units may be performed by separate hardware, firmware, or software components, or integrated within common or separate hardware, firmware, or software components.

The disclosure contemplates computer-readable storage media comprising instructions to cause a processor to perform any of the functions and techniques described herein. The computer-readable storage media may take the example form of any volatile, non-volatile, magnetic, optical, or electrical media, such as a RAM, ROM, NVRAM, EEPROM, or flash memory that is tangible. The computer-readable storage media may be referred to as non-transitory. A server, client computing device, or any other computing device may also contain a more portable removable memory type to enable easy data transfer or offline data analysis.

The techniques described in this disclosure, including those attributed to various circuitry and various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated, discrete logic circuitry, or other processor circuitry, as well as any combinations of such components, remote servers, remote client devices, or other devices. The term "processor circuitry" or "processor circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. In addition, any of the described units, circuitry or components may be implemented together or separately as discrete but interoperable logic devices. Depiction of different features as circuitry or units is intended to highlight different functional aspects and does not necessarily imply that such circuitry or units must be realized by separate hardware or software components. Rather, functionality associated with one or more circuitry or units may be performed by separate hardware or software components, or integrated within common or separate hardware or software components. For example, any circuitry described herein may include electrical circuitry configured to perform the features attributed to that particular circuitry, such as fixed function processor circuitry, programmable processor circuitry, or combinations thereof.

The techniques described in this disclosure may also be embodied or encoded in an article of manufacture including a computer-readable storage medium encoded with instructions. Instructions embedded or encoded in an article of manufacture including a computer-readable storage medium encoded, may cause one or more programmable processors, or other processors, to implement one or more of the techniques described herein, such as when instructions included or encoded in the computer-readable storage medium are executed by the one or more processors. Example computer-readable storage media may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or any other computer readable storage devices or tangible computer readable media. The computer-readable storage medium may also be referred to as storage devices.

In some examples, a computer-readable storage medium comprises non-transitory medium. The term "non-transitory" may indicate that the storage medium is not embodied in a carrier wave or a propagated signal. In certain examples, a non-transitory storage medium may store data that may, over time, change (e.g., in RAM or cache).

Various examples have been described herein. Any combination of the described operations or functions is contemplated. These and other examples are within the scope of the following claims. Based upon the above discussion and illustrations, it is recognized that various modifications and changes may be made to the disclosed examples in a manner that does not require strictly adherence to the examples and applications illustrated and described herein. Such modifications do not depart from the true spirit and scope of various aspects of disclosure. The invention is set out in the claims that follow.

## Claims

1. An implantable neurostimulator comprising:
a battery configured to provide power to the implantable neurostimulator;
a battery switch (72) configured to open and remove power from one or more components of the implantable neurostimulator, or close to provide power to each component of the implantable neurostimulator requiring power to operate; and
processing circuitry configured to:
execute a plurality of firmware modules that are configured to perform respective functions of the implantable neurostimulator, at least two of the plurality of firmware modules being configured to determine whether power is needed for a corresponding hardware component or to perform the respective function during a respective time period and to generate and transmit one or more respective requests based on the determination of whether power is needed for the corresponding hardware component or to perform the respective function during the respective time period; and
execute power domain firmware that configures the processing circuitry to:
receive the one or more respective requests;
determine whether to open the battery switch (72) or to keep the battery switch (72) closed in response to the one or more respective requests; and
control the battery switch (72) to open or keep closed in response to the determination.

2. The implantable neurostimulator of claim 1, wherein the one or more respective requests comprise at least one of:
a request to allow the battery switch (72) to be opened at any time until further notice;
a request to prevent the battery switch (72) from being opened until further notice;
a request to prevent the battery switch (72) from being opened until a first time period expires; or
a request to allow the battery switch (72) to be opened now, but to require the battery switch (72) to be closed when a second time period expires.

3. The implantable neurostimulator of claim 1 or 2, wherein the one or more respective requests each permit the battery switch (72) to be opened, and wherein the determination is to open the battery switch (72).

4. The implantable neurostimulator of claim 3, wherein therapy delivery is in an off-cycle.

5. The implantable neurostimulator of any of claims 1-4, wherein at least one of the one or more respective requests comprises a request to prevent the battery switch (72) from opening, and wherein the determination is to keep the battery switch (72) closed.

6. The implantable neurostimulator of any of claims 1-5, wherein the power domain firmware further configures the processing circuitry to control the battery switch (72) to open only during a given time period when each of the plurality of firmware modules have not requested that the battery switch (72) be prevented from being opened during the given time period.

7. The implantable neurostimulator of any of claims 1-6, wherein the plurality of firmware modules comprise at least one of:
non-volatile memory firmware;
device time firmware;
fuel gauge firmware;
device recharger firmware;
therapy delivery firmware;
telemetry firmware; or
sensing firmware.

8. The implantable neurostimulator of any of claims 1-7, further comprising:
stimulation circuitry configured to generate an electrical stimulation signal; and
telemetry circuitry configured to communicate with another device.

9. The implantable neurostimulator of any of claims 1-8, wherein the power domain firmware further configures the processing circuitry to store, in memory, a lifetime count of a cumulative amount of time the battery switch (72) is closed and a lifetime count of a cumulative amount of time the battery switch (72) is open.

10. The implantable neurostimulator of any of claims 1-9, wherein the one or more respective requests comprise one or more interrupts.

11. A non-transitory computer-readable storage medium comprising instructions for processing circuitry of an implantable neurostimulator comprising a battery configured to provide power to the implantable neurostimulator and a battery switch (72) configured to open and remove power from one or more components of the implantable neurostimulator, or close to provide power to each component of the implantable neurostimulator requiring power to operate, the instructions including a plurality of firmware modules and a power domain firmware, the instructions, when executed, cause the processing circuitry of the implantable neurostimulator to:
perform respective functions of the implantable neurostimulator;
determine, by at least two of the plurality of firmware modules executing on the processing circuitry, whether power is needed for a corresponding hardware component or to perform the respective function during a respective time period;
generate and transmit, by the at least two of the plurality of firmware modules executing on the processing circuitry, one or more respective requests based on the determination of whether power is needed for the corresponding hardware component or to perform the respective function during the respective time period;
receive, by the power domain firmware executing on the processing circuitry, the one or more respective requests;
determine, by the power domain firmware executing on the processing circuitry, whether to open the battery switch (72) or to keep the battery switch (72) closed in response to the one or more respective requests; and control the battery switch (72) to open or keep closed in response to the determination.

12. The non-transitory computer-readable storage medium of claim 11, wherein the one or more respective requests comprising at least one of:
a request to allow the battery switch to be opened at any time until further notice;
a request to prevent the battery switch from being opened until further notice;
a request to prevent the battery switch from being opened until a first time period expires; or
a request to allow the battery switch to be opened now, but to require the battery switch to be closed when a second time period expires.

13. The non-transitory computer-readable storage medium of claim 11 or 12, wherein the one or more respective requests each permit the battery switch to be opened and wherein the determination is to open the battery switch.

14. The non-transitory computer-readable storage medium of claim 13, wherein therapy delivery is in an off-cycle.

15. A method comprising:
receiving, by power domain firmware executing on processing circuitry of the implantable neurostimulator of any of claims 1-10 and from at least two of a plurality of firmware modules executing on the processing circuitry and configured to determine whether power is needed for a respective corresponding hardware component or to perform a respective function of the implantable neurostimulator of any of claims 1-10 during a respective time period and to generate and transmit one or more respective requests based on the determination of whether power is needed for the corresponding hardware component or to perform the respective function during the respective time period, the one or more respective requests;
determining, by the power domain firmware, whether to open the battery switch (72) of the implantable neurostimulator of any of claims 1-10 or to keep the battery switch (72) closed in response to the one or more respective requests; and
controlling, by the power domain firmware, the battery switch (72) to open or keep closed in response to the determination.

## Patentansprüche

1. Implantierbarer Neurostimulator, umfassend:
eine Batterie, die konfiguriert ist, um den implantierbaren Neurostimulator mit Strom zu versorgen;
einen Batterieschalter (72), der konfiguriert ist, um sich zu öffnen und eine oder mehrere Komponenten des implantierbaren Neurostimulators vom Strom zu trennen oder zu schließen, um jede Komponente des implantierbaren Neurostimulators, die Strom benötigt, um betrieben zu werden, mit Strom zu versorgen; und
eine Verarbeitungsschaltlogik, die konfiguriert ist zum:
Ausführen einer Vielzahl von Firmwaremodulen, die konfiguriert ist, um jeweilige Funktionen des implantierbaren Neurostimulators durchzuführen, wobei mindestens zwei der Vielzahl von Firmwaremodulen konfiguriert sind, um zu bestimmen, ob Strom für eine entsprechende Hardwarekomponente oder um die jeweilige Funktion während eines jeweiligen Zeitraums durchzuführen benötigt wird, und um eine oder mehrere jeweilige Anforderungen basierend auf der Bestimmung zu erzeugen und übertragen, ob Strom für die entsprechende Hardwarekomponente oder um die jeweilige Funktion während des jeweiligen Zeitraums durchzuführen benötigt wird; und
Ausführen der Stromdomänen-Firmware, die die Verarbeitungsschaltung konfiguriert zum:
Empfangen der einen oder der mehreren jeweiligen Anfragen;
Bestimmen, als Reaktion auf die eine oder die mehreren jeweiligen Anforderungen, ob der Batterieschalter (72) geöffnet werden soll oder ob der Batterieschalter (72) geschlossen gehalten werden soll; und
Steuern des Batterieschalters (72), um sich, als Reaktion auf die Bestimmung, zu öffnen oder geschlossen zu halten.

2. Implantierbarer Neurostimulator nach Anspruch 1, wobei die eine oder die mehreren jeweiligen Anforderungen mindestens eines umfassen von:
einer Anforderung, um dem Batterieschalter (72) zu erlauben, zu einer beliebigen Zeit bis auf Weiteres geöffnet zu werden;
einer Anforderung, um den Batterieschalter (72) daran zu hindern, bis auf Weiteres geöffnet zu werden;
einer Anforderung, um den Batterieschalter (72) daran zu hindern, geöffnet zu werden, bis eine erste Zeitspanne abläuft; oder
einer Anforderung, um dem Batterieschalter (72) zu erlauben, jetzt geöffnet zu werden, von dem Batterieschalter (72) jedoch zu fordern, geschlossen zu werden, wenn eine zweite Zeitspanne abgelaufen ist.

3. Implantierbarer Neurostimulator nach Anspruch 1 oder 2, wobei die eine oder die mehreren jeweiligen Anforderungen dem Batterieschalter (72) jeweils gestatten, geöffnet zu werden, und wobei die Bestimmung darin besteht, den Batterieschalter (72) zu öffnen.

4. Implantierbarer Neurostimulator nach Anspruch 3, wobei eine Therapieabgabe in einem Aus-Zyklus erfolgt.

5. Implantierbarer Neurostimulator nach einem der Ansprüche 1 bis 4, wobei mindestens eine der einen oder der mehreren jeweiligen Anforderungen eine Anforderung umfasst, den Batterieschalter (72) daran zu hindern, sich zu öffnen, und wobei die Bestimmung darin besteht, den Batterieschalter (72) geschlossen zu halten.

6. Implantierbarer Neurostimulator nach einem der Ansprüche 1 bis 5, wobei die Stromdomänen-Firmware die Verarbeitungsschaltung ferner konfiguriert, um den Batterieschalter (72) zu steuern, um sich nur während eines gegebenen Zeitraums zu öffnen, wenn jedes der Vielzahl von Firmwaremodulen nicht angefordert hat, dass der Batterieschalter (72) daran gehindert werden soll, während des gegebenen Zeitraums geöffnet zu werden.

7. Implantierbarer Neurostimulator nach einem der Ansprüche 1 bis 6, wobei die Vielzahl von Firmwaremodulen mindestens eines umfasst von:
Firmware eines nichtflüchtigen Speichers;
Vorrichtungszeit-Firmware;
Tankanzeigen-Firmware;
Vorrichtungsaufladegerät-Firmware;
Therapieabgabe-Firmware;
Telemetrie-Firmware; oder
Sensor-Firmware.

8. Implantierbarer Neurostimulator nach einem der Ansprüche 1 bis 7, ferner umfassend: eine Stimulationsschaltung, die konfiguriert ist, um ein elektrisches Stimulationssignal zu erzeugen; und
Telemetrieschaltung, die konfiguriert ist, um mit einer anderen Vorrichtung zu kommunizieren.

9. Implantierbarer Neurostimulator nach einem der Ansprüche 1 bis 8, wobei die Stromdomänen-Firmware die Verarbeitungsschaltung ferner konfiguriert, um, in einem Speicher, einen Lebensdauerzähler einer kumulierten Zeitdauer, in der der Batterieschalter (72) geschlossen ist, und einen Lebensdauerzähler einer kumulierten Zeitdauer, in der der Batterieschalter (72) geöffnet ist, zu speichern.

10. Implantierbarer Neurostimulator nach einem der Ansprüche 1 bis 9, wobei die eine oder die mehreren jeweiligen Anforderungen eine oder mehrere Unterbrechungen umfassen.

11. Nicht flüchtiges, computerlesbares Speicherungsmedium, umfassend Anweisungen für die Verarbeitungsschaltung eines implantierbaren Neurostimulators, umfassend eine Batterie, die konfiguriert ist, um den implantierbaren Neurostimulator mit Strom zu versorgen, und einen Batterieschalter (72), der konfiguriert ist, um eine oder mehrere Komponenten des implantierbaren Neurostimulators zu öffnen und vom Strom zu trennen, oder zu schließen, um jede Komponente des implantierbaren Neurostimulators, die Strom benötigt, um betrieben zu werden, mit Strom zu versorgen, wobei die Anweisungen eine Vielzahl von Firmwaremodulen und eine Stromdomänen-Firmware einschließen, wobei die Anweisungen, wenn sie ausgeführt werden, die Verarbeitungsschaltung des implantierbaren Neurostimulators veranlassen zum:
Durchführen der jeweiligen Funktionen des implantierbaren Neurostimulators;
Bestimmen, durch mindestens zwei der Vielzahl von Firmwaremodulen, die auf der Verarbeitungsschaltung ausgeführt werden, ob Strom für eine entsprechende Hardwarekomponente oder um die jeweilige Funktion während eines jeweiligen Zeitraums durchzuführen benötigt wird;
Erzeugen und Übertragen, durch die mindestens zwei der Vielzahl von Firmwaremodulen, die auf der Verarbeitungsschaltung ausgeführt werden, einer oder mehrerer jeweiliger Anforderungen basierend auf der Bestimmung, ob Strom für die entsprechende Hardwarekomponente oder um die jeweilige Funktion während des jeweiligen Zeitraums auszuführen benötigt wird;
Empfangen, durch die Stromdomänen-Firmware, die auf der Verarbeitungsschaltung ausgeführt wird, der einen oder der mehreren jeweiligen Anforderungen;
Bestimmen, durch die Stromdomänen-Firmware, die auf der Verarbeitungsschaltung ausgeführt wird, als Reaktion auf die eine oder die mehreren jeweiligen Anforderungen, ob der Batterieschalter (72) geöffnet werden soll oder ob der Batterieschalter (72) geschlossen gehalten werden soll; und Steuern des Batterieschalters (72), um sich als Reaktion auf die Bestimmung zu öffnen oder geschlossen zu halten.

12. Nicht flüchtiges computerlesbares Speicherungsmedium nach Anspruch 11, die eine oder die mehreren jeweiligen Anforderungen umfassend mindestens eines von:
der Anforderung, dem Batterieschalter zu erlauben, zu einer beliebigen Zeit bis auf Weiteres geöffnet zu werden;
einer Anforderung, den Batterieschalter daran zu hindern, bis auf Weiteres geöffnet zu werden;
einer Anforderung, den Batterieschalter daran zu hindern, geöffnet zu werden, bis eine erste Zeitspanne aubgelaufen ist; oder
einer Anforderung, dem Batterieschalter zu erlauben, jetzt geöffnet zu werden, von dem Batterieschalter jedoch zu fordern, geschlossen zu werden, wenn eine zweite Zeitspanne abgelaufen ist.

13. Nicht flüchtiges, computerlesbares Speicherungsmedium nach Anspruch 11 oder 12, wobei die eine oder die mehreren jeweiligen Anforderungen dem Batterieschalter jeweils gestatten, geöffnet zu werden, und wobei die Bestimmung darin besteht, den Batterieschalter zu öffnen.

14. Nichtflüchtiges, computerlesbares Speicherungsmedium nach Anspruch 13, wobei die Therapieabgabe in einem Aus-Zyklus erfolgt.

15. Verfahren, umfassend:
Empfangen, durch Energiedomänen-Firmware, die auf der Verarbeitungsschaltung des implantierbaren Neurostimulators nach einem der Ansprüche 1 bis 10 ausgeführt wird, und von mindestens zwei einer Vielzahl von Firmwaremodulen, die auf der Verarbeitungsschaltung ausgeführt werden und konfiguriert sind, um zu bestimmen, ob Strom für eine jeweilige entsprechende Hardwarekomponente oder um eine jeweilige Funktion des implantierbaren Neurostimulators nach einem der Ansprüche 1 bis 10 während eines jeweiligen Zeitraums durchzuführen benötigt wird, und um eine oder mehrere jeweilige Anforderungen basierend auf der Bestimmung, ob Energie für die entsprechende Hardwarekomponente oder um die jeweilige Funktion während des jeweiligen Zeitraums durchzuführen benötigt wird, zu erzeugen und zu übertragen, der einen oder der mehreren jeweiligen Anforderungen;
Bestimmen, durch die Stromdomänen-Firmware, als Reaktion auf die eine oder die mehreren jeweiligen Anforderungen, ob der Batterieschalter (72) des implantierbaren Neurostimulators nach einem der Ansprüche 1 bis 10 geöffnet werden soll oder ob der Batterieschalter (72) geschlossen gehalten werde soll; und Steuern, durch die Stromdomänen-Firmware, des Batterieschalters (72) um sich als Reaktion auf die Bestimmung zu öffnen oder geschlossen zu halten.

## Revendications

1. Neurostimulateur implantable, comprenant :
une batterie configurée pour alimenter le neurostimulateur implantable ;
un interrupteur de batterie (72) configuré pour s'ouvrir et couper l'alimentation d'un ou de plusieurs composants du neurostimulateur implantable, ou se fermer pour alimenter chaque composant du neurostimulateur implantable nécessitant une alimentation pour fonctionner ; et
des circuits de traitement configurés pour :
exécuter une pluralité de modules de micrologiciel qui sont configurés pour exécuter des fonctions respectives du neurostimulateur implantable, au moins deux parmi la pluralité de modules de micrologiciel étant configurés pour déterminer si une alimentation est nécessaire pour un composant matériel correspondant ou pour exécuter la fonction respective pendant un laps de temps donné et pour générer et transmettre une ou plusieurs requêtes respectives en fonction de la détermination de la nécessité d'une alimentation pour le composant matériel correspondant ou pour exécuter la fonction respective pendant le laps de temps donné ; et
exécuter un micrologiciel de domaine de puissance qui configure le circuit de traitement pour :
recevoir la ou les demandes respectives ;
déterminer s'il faut ouvrir l'interrupteur de batterie (72) ou maintenir l'interrupteur de batterie (72) fermé en réponse à la ou les demandes respectives ; et
commander l'interrupteur de batterie (72) pour qu'il s'ouvre ou reste fermé en réponse à la détermination.

2. Neurostimulateur implantable selon la revendication 1, dans lequel la ou les requêtes respectives comprennent au moins l'une parmi :
une requête pour permettre à l'interrupteur de batterie (72) d'être ouvert à tout moment jusqu'à nouvel ordre ;
une requête visant à empêcher l'interrupteur de batterie (72) d'être ouvert jusqu'à nouvel ordre ;
une requête visant à empêcher l'interrupteur de batterie (72) d'être ouvert jusqu'à l'expiration d'un premier délai ; ou
une requête pour permettre à l'interrupteur de batterie (72) d'être ouvert maintenant, mais nécessitant que l'interrupteur de batterie (72) soit fermé à l'expiration d'un second laps de temps.

3. Neurostimulateur implantable selon la revendication 1 ou 2, dans lequel le ou les requêtes respectives permettent chacune à l'interrupteur de batterie (72) d'être ouvert, et dans lequel la détermination est d'ouvrir l'interrupteur de batterie (72).

4. Neurostimulateur implantable selon la revendication 3, dans lequel la thérapie est administrée en dehors du cycle.

5. Neurostimulateur implantable selon l'une quelconque des revendications 1 à 4, dans lequel au moins l'une parmi la ou les requêtes respectives comprend une requête visant à empêcher l'ouverture de l'interrupteur de batterie (72), et dans lequel la détermination est de maintenir l'interrupteur de batterie (72) fermé.

6. Neurostimulateur implantable selon l'une quelconque des revendications 1 à 5, dans lequel le micrologiciel du domaine de puissance configure en outre le circuit de traitement pour commander l'ouverture de l'interrupteur de batterie (72) uniquement pendant un laps de temps donné lorsque chacune de la pluralité de modules du micrologiciel n'a pas demandé que l'on empêche l'ouverture de l'interrupteur de batterie (72) pendant le laps de temps donné.

7. Neurostimulateur implantable selon l'une quelconque des revendications 1 à 6, dans lequel la pluralité de modules de micrologiciel comprend au moins l'un parmi :
un micrologiciel à mémoire non volatile ;
un micrologiciel de l'heure de l'appareil ;
un micrologiciel de jauge de carburant ;
un micrologiciel de recharge de l'appareil ;
un micrologiciel d'administration de thérapie ;
un micrologiciel de télémétrie ; ou
un micrologiciel de détection.

8. Neurostimulateur implantable selon l'une quelconque des revendications 1 à 7 comprenant en outre : un circuit de stimulation configuré pour générer un signal de stimulation électrique ; et
un circuit de télémétrie configuré pour communiquer avec un autre appareil.

9. Neurostimulateur implantable selon l'une quelconque des revendications 1 à 8, dans lequel le micrologiciel du domaine de puissance configure en outre le circuit de traitement pour stocker, dans la mémoire, un décompte de la durée de vie d'une quantité cumulative de temps pendant lequel l'interrupteur de batterie (72) est fermé et un décompte de la durée de vie d'une quantité cumulative de temps pendant lequel l'interrupteur de batterie (72) est ouvert.

10. Neurostimulateur implantable selon l'une quelconque des revendications 1 à 9, dans lequel la ou les requêtes respectives comprennent une ou plusieurs interruptions.

11. Support de stockage non transitoire lisible par ordinateur comprenant des instructions destinées au traitement de circuit d'un neurostimulateur implantable comprenant une batterie configurée pour alimenter le neurostimulateur implantable et un interrupteur de batterie (72) configuré pour s'ouvrir et couper l'alimentation d'un ou plusieurs composants du neurostimulateur implantable, ou se fermer pour alimenter chaque composant du neurostimulateur implantable nécessitant une alimentation pour fonctionner, les instructions comportant une pluralité de modules de micrologiciel et un micrologiciel de domaine de puissance, les instructions, lorsqu'elles sont exécutées, amènent le circuit de traitement du neurostimulateur implantable à :
exécuter les fonctions respectives du neurostimulateur implantable ;
déterminer, par au moins deux de la pluralité de modules de micrologiciel s'exécutant sur le circuit de traitement, si une alimentation est nécessaire pour un composant matériel correspondant ou pour exécuter la fonction respective pendant un laps de temps respectif ;
générer et transmettre, par au moins deux de la pluralité de modules de micrologiciel s'exécutant sur le circuit de traitement, une ou plusieurs requêtes respectives en fonction de la détermination de la nécessité d'alimenter le composant matériel correspondant ou d'exécuter la fonction respective pendant le laps de temps respectif ;
recevoir, par le micrologiciel du domaine de puissance s'exécutant sur le circuit de traitement, le ou les requêtes respectives ;
déterminer, par le micrologiciel du domaine de puissance s'exécutant sur le circuit de traitement, s'il faut ouvrir l'interrupteur de batterie (72) ou maintenir l'interrupteur de batterie (72) fermé en réponse à la ou les requêtes respectives ; et commander l'interrupteur de batterie (72) pour qu'il s'ouvre ou reste fermé en réponse à la détermination.

12. Support de stockage non transitoire lisible par ordinateur selon la revendication 11, dans lequel la ou les requêtes respectives comprennent au moins l'une parmi :
une requête pour permettre à l'interrupteur de batterie d'être ouvert à tout moment jusqu'à nouvel ordre ;
une requête visant à empêcher l'ouverture de l'interrupteur de batterie jusqu'à nouvel ordre ;
une requête visant à empêcher l'ouverture de l'interrupteur de batterie jusqu'à l'expiration d'un premier délai ; ou
une requête pour permettre à l'interrupteur de batterie d'être ouvert maintenant, mais nécessitant que l'interrupteur de batterie soit fermé à l'expiration d'un second laps de temps.

13. Support de stockage non transitoire lisible par ordinateur selon la revendication 11 ou 12, dans lequel le ou les requêtes respectives permettent chacune l'ouverture de l'interrupteur de batterie et dans lequel la détermination est d'ouvrir l'interrupteur de batterie.

14. Support de stockage non transitoire lisible par ordinateur selon la revendication 13, dans lequel l'administration de la thérapie se fait hors cycle.

15. Procédé comprenant :
la réception, par le micrologiciel du domaine de puissance s'exécutant sur le circuit de traitement du neurostimulateur implantable selon l'une quelconque des revendications 1 à 10 et d'au moins deux modules d'une pluralité de modules de micrologiciel s'exécutant sur le circuit de traitement et configurés pour déterminer si une alimentation est nécessaire pour un composant matériel correspondant respectif ou pour exécuter une fonction respective du neurostimulateur implantable selon l'une quelconque des revendications 1 à 10 pendant un laps de temps respectif et pour générer et transmettre une ou plusieurs requêtes respectives en fonction de la détermination de la nécessité d'une alimentation pour le composant matériel correspondant ou pour exécuter la fonction respective pendant le laps de temps respectif, le ou les requêtes respectives ;
la détermination, par le micrologiciel du domaine d'alimentation, s'il faut ouvrir l'interrupteur de batterie (72) du neurostimulateur implantable selon l'une quelconque des revendications 1 à 10 ou maintenir l'interrupteur de batterie (72) fermé en réponse à la ou les requêtes respectives ; et la commande, par le micrologiciel du domaine d'alimentation, d'ouvrir ou de maintenir fermé l'interrupteur de batterie (72) en réponse à la détermination.
